# EUROPEAN PATENT APPLICATION

(11) **EP 3 628 664 A1**
(43) Date of publication of application: **01.04.2020**
(21) Application number: 18196711.8
(22) Date of filing: 25.09.2018
(51) Int. Cl.: C07D 239/30, C07D 241/24, A61P 35/00, A61K 31/506, A61K 31/497

(54) **IRREVERSIBLE INHIBITORS OF KRAS G12C MUTANT**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to novel substituted 2-halopyrimidines and 5-halopyrazines, as well as pharmaceutical compositions containing at least one of these substituted 2-halopyrimidines or 5-halopyrazines together with at least one pharmaceutically acceptable carrier, excipient and/or diluent. Said substituted 2-halopyrimidines and 5-halopyrazines are covalently binding to KRas mutant G12C and therefore, are useful for the prophylaxis and treatment of cancer by direct inhibition of the KRas G12C-oncogenic signaling in cells.

## Description

### Field of the invention

The present invention relates to novel substituted 2-halopyrimidines and 5-halopyrazines, as well as pharmaceutical compositions containing at least one of these substituted 2-halopyrimidines or 5-halopyrazines together with at least one pharmaceutically acceptable carrier, excipient and/or diluent. Said substituted 2-halopyrimidines and 5-halopyrazines are covalently binding to the KRas mutant G12C and therefore, are useful for the prophylaxis and treatment of cancer by direct inhibition of the KRas G12C-ongogenic signaling in cells.

### Background of the invention

In cancer treatment, there is an ongoing need for the development of novel substances, which are effective to induce cell cycle/proliferation arrest or cell death of cancer cells. The fundamental characteristics of these cells are that the control of the cell cycle and proliferation is disturbed and they evade oncogene induced stress response and cell senescence.

KRas as well as HRas and NRas, which belong to the Ras proteins are key regulators of diverse cellular processes including proliferation and differentiation. The KRas protein is normally tightly regulated by guanine nucleotide exchange factors (GEFs) promoting GDP dissociation and GTP binding and GTPase-activating proteins (GAPs) that stimulate the intrinsic GTPase activity of KRas to switch off signaling. KRas alternates between an active "on" state with a bound GTP and an inactive "off" state with a bound GDP. The active "on" state binds to and activates proteins that control growth and differentiation of cells. Aberrant KRas function is associated with proliferative disorders, cancers and tumors. Mutation at these conserved sites favors GTP binding and produces constitutive activation of KRas.

20 to 30% of the human tumors have activating point mutations in Ras, most frequently in KRas, followed by NRas, then HRas. These mutations all compromise the GTPase activity of Ras, preventing GAPs from promoting hydrolysis of GTP on Ras and therefore, causing Ras to accumulate in the GTP-bound, active form. Despite substantial efforts to interfere with signaling by oncogenic Ras proteins, in particular by means of Ras-farnesyltransferase inhibitors, up to present no clinically useful drug has been found. The KRas mutations are very frequent in lung, colorectal and pancreatic cancer, wherein the KRas G12C point mutation is most common in lung cancer and often results from a G-to-T transversion caused by carcinogens in cigarette smoke (Fut. Oncol. 2017, 13, 263-271).

The oncogenic KRas G12C mutation is a promising target which led to the design of direct covalent inhibitors due to the presence of the unique nucleophilic cysteine residue that allows covalent targeting by electrophilic small molecules and due to the absence of the mutation in normal tissue.

The international patent application WO 2014/152588 A1 discloses irreversible piperazine and/or azetidine containing KRas G12C inhibitors that bind at Cys12 in the switch II via an acrylamide group. The international patent application WO 2015/054572 A1 also discloses irreversible KRAS G12C inhibitors that bind to Cys12 via an acrylamide group. A similar inhibitor ARS-853, which also contains an acrylamide warhead is disclosed in Cancer Discov. 2016 Mar;6(3):316-29. A different KRAS G12C inhibitor is described by Hunter et al. (PNAS 2014, 111, 8895), wherein the GDP derivative SML-8-73-1 acts as a GTP-competitive inhibitor and reacts with the thiol group of Cys12 via a chloroacetamide. However, these inhibitors are of limited use in vivo due to limited cell stability, permeability and low cellular activity. Most allele-specific KRas G12C covalent binders make use of an unstable and unselective acrylamide warhead, therefore the development of other electrophilic warheads that can be attached to KRas ligands and that display a high stability and selective KRas G12C covalent labeling are on high demand.

It is the objective of the present invention to provide irreversible Inhibitors of KRas G12C that can be used as pharmaceutically active agents, especially for the treatment or prophylaxis of cancers, tumors and proliferative diseases, as well as compositions comprising at least one of these compounds as pharmaceutically active agent.

The objective of the present invention is solved by the teaching of the independent claims. Further advantageous features, aspects and details of the invention are evident from the dependent claims, the description, the figures, and the examples of the present application.

### Description of the invention

It was surprisingly found that 2-halopyrimidines and 5-halopyrazines of general formula (I) bind highly selectively and covalently to cysteine in the Switch II pocket of KRas G12C and suppress the activation of KRas and oncogenic KRas signaling in cells, thereby causing inhibition of cell proliferation and cell death of KRas dependent cell lines. Hence, these inventive compounds are useful for the treatment or prophylaxis of cancers, tumors and other proliferative diseases.

Thus, the present invention refers to a compound of general formula (I) 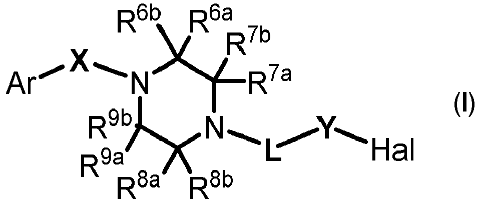 wherein
Ar represents 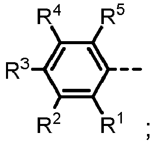
**R¹** - **R⁵** represent independently of each other -H, -F, -Cl, -CN, -CF₃, -OCF₃, -CH₃, -C₂H₅, -C₃H₇, -C₄H₉, -CH(CH₃)₂, -CH₂CH(CH₃)₂, -CH(CH₃)-C₂H₅, -C(CH₃)₃, -C(CH₃)R¹⁰R¹¹, -*cyclo*-C₃H₅, *-cyclo-*C₄H₇, -*cyclo*-C₅H₉, -CH₂-*cyclo*-C₃H₅, -Ph, -OH, 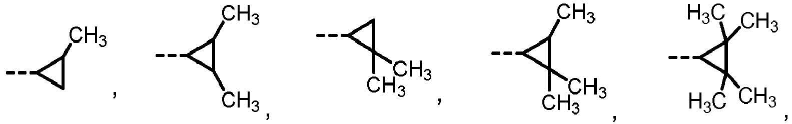 -OCH₃, -OC₂H₅, -OC₃H₇, -OC₄H₉, -OCH(CH₃)₂, -OCH₂CH(CH₃)₂, -OCH(CH₃)-C₂H₅, -OC(CH₃)₃, -O-*cyclo*-C₃H₅, -OCH₂-*cyclo*-C₃H₅, -NH₂, -NH(CH₃), -NH(C₂H₅), -N(CH₃)₂, -N(C₂H₅)₂ or -N(C₃H₇)₂,
**R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -O-cyclo-C₃H₅, -OCH₂-cyclo-C₃H₅, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -NHCOCH₃, -NHCOCF₃, -CONH₂, -CONHCH₃, -CONHC₂H₅, -CON(CH₃)₂, -CON(C₂H₅)₂, -SO₂NH₂, -SO₂NHCH₃, -SO₂NHC₂H₅, -SO₂N(CH₃)₂, -SO₂N(C₂H₅)₂, -NHSO₂CH₃, -NHSO₂CF₃ or -NHSO₂C₂H₅; or
**R^{6a}** and **R^{7a}** join to form a 5-membered or 6-membered carbocyclic or heterocyclic ring, or **R^{6b}** and **R^{7b}** join to form a 5-membered or 6-membered carbocyclic or heterocyclic ring, or **R^{8a}** and **R^{9a}** join to form a 5-membered or 6-membered carbocyclic or heterocyclic ring, or **R^{8b}** and **R^{9b}** join to form a 5-membered or 6-membered carbocyclic or heterocyclic ring;
**R¹⁰** and **R¹¹** represent independently of each other -CH₃, -CH₂CH₃, -CH₂CH₂CH₃, -CH(CH₃)₂, or **R¹⁰** and **R¹¹** together with carbon atom to which they are attached form a cyclopropane, cyclobutane or cyclopentane ring;
**L** represents -C(O)- or -SO₂-;
**X** represents -NH-CH₂-CO- or -O-CH₂-CO-;
**Y** represents 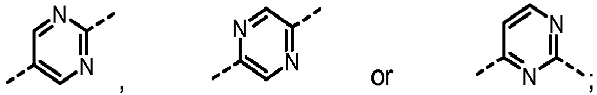
**Hal** represents -Cl, -Br or -F;
or pharmaceutically acceptable salts thereof.

Preferably, **R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -CONH₂, -CONHCH₃, -CONHC₂H₅, -CON(CH₃)₂ or -CON(C₂H₅)₂. Thus, the present invention is also directed to a compound of general formula (I), wherein **R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -CONH₂, -CONHCH₃, -CONHC₂H₅, -CON(CH₃)₂ or -CON(C₂H₅)₂.

Preferably, **R^{6a}** and **R^{7a}** join to form a 5-membered or 6-membered carbocyclic or heterocyclic ring, or **R^{6b}** and **R^{7b}** join to form a 5-membered or 6-membered carbocyclic or heterocyclic ring, or **R^{8a}** and **R^{9a}** join to form a 5-membered or 6-membered carbocyclic or heterocyclic ring, or **R^{8b}** and **R^{9b}** join to form a 5-membered or 6-membered carbocyclic or heterocyclic ring, wherein the 5-membered or 6-membered carbocyclic or heterocyclic ring formed between **R^{6a}** and **R^{7a}**, **R^{6b}** and **R^{7b}**, **R^{8a}** and **R^{9a}** and **R^{8b}** and **R^{9b}** is independently selected from: 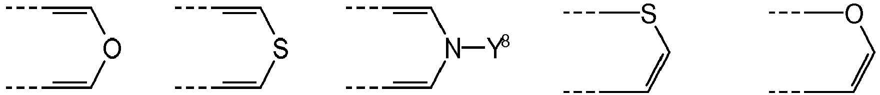 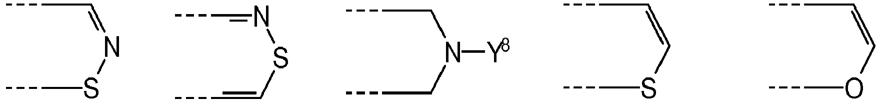 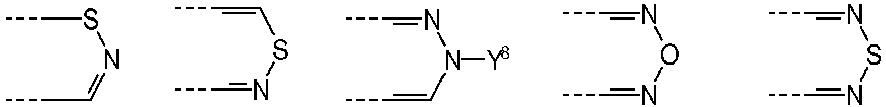 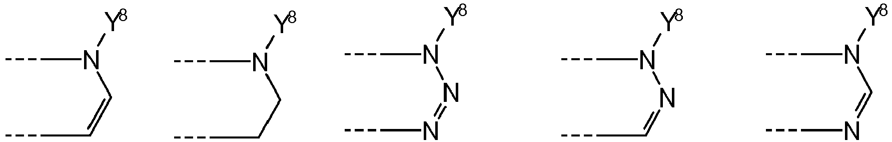 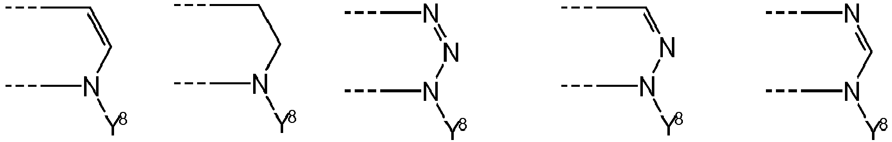 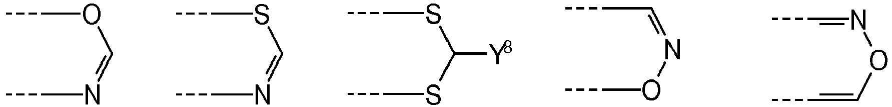 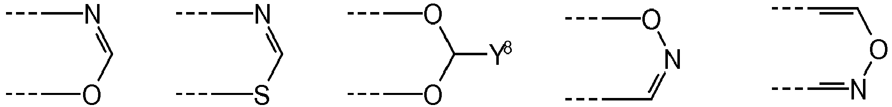 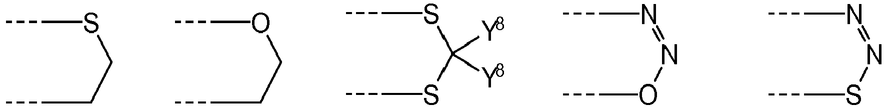 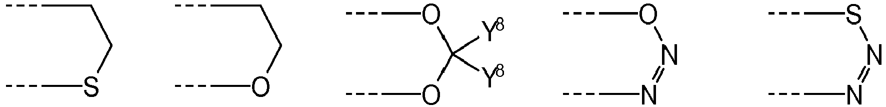 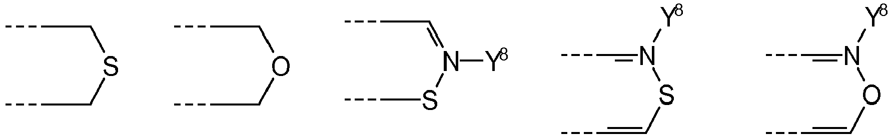 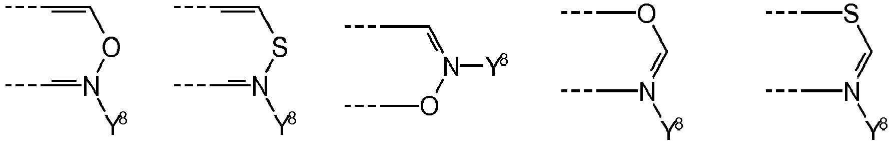 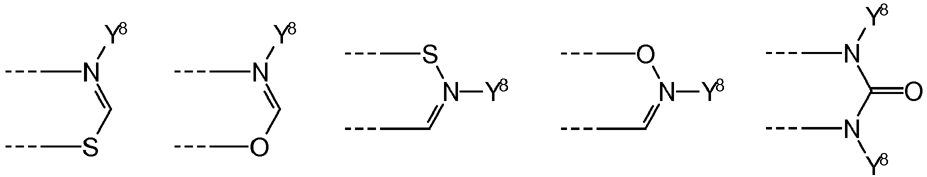 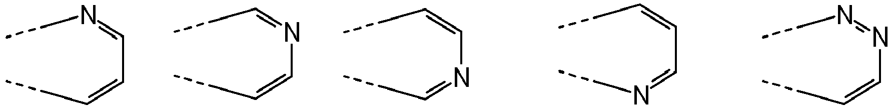 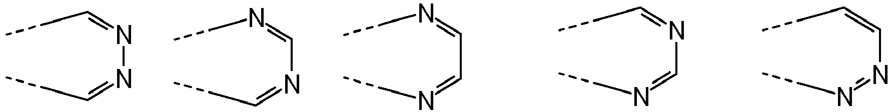 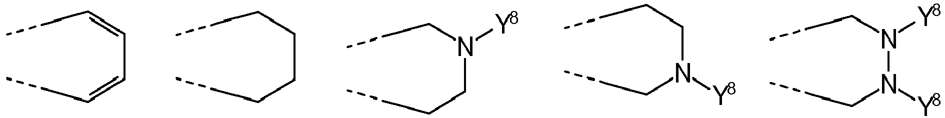 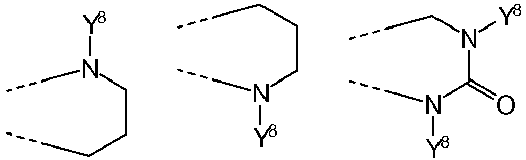 wherein Y⁸ represents -H, -CH₃, -C₂H₅, -C₃H₇, -C₄H₉, -CH(CH₃)₂, -CH₂CH(CH₃)₂, -CH(CH₃)-C₂H₅, -C(CH₃)₃ or -cyclo-C₃H₅.

More preferably, **R^{6a}** - **R^{9a}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)_{2,} -N(C₂H₅)₂, -CH₂NH₂, -CH₂NHCH₃, -CH₂NHC₂H₅, -CH₂N(CH₃)₂, -CH₂N(C₂H₅)₂, -CONH₂, -CONHCH₃, -CONHC₂H₅, -CON(CH₃)₂ or -CON(C₂H₅)₂; and **R^{6b}** - **R^{9b}** represent -H.

Most preferably **R^{6a}** - **R^{9a}** and **R^{6b}** - **R^{9b}** represent -H. Thus, the present invention is also directed to a compound of general formula (I), wherein **R^{6a}** - **R^{9a}** and **R^{6b} - R^{9b}** represent -H.

In a preferred embodiment, **X** represents -NH-CH₂-CO- . In a further preferred embodiment, **Hal** represents -Cl. Particularly preferred is, when **X** represents -NH-CH₂-CO- and **Hal** represents -Cl.

One embodiment of the present invention is directed to a compound of general formula (I), wherein
Ar represents 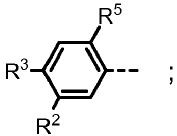 **R²** represents -C(CH₃)R¹⁰R¹¹, -*cyclo*-C₃H₅, *-cyclo-*C₄H₇, -*cyclo*-C₅H₉, 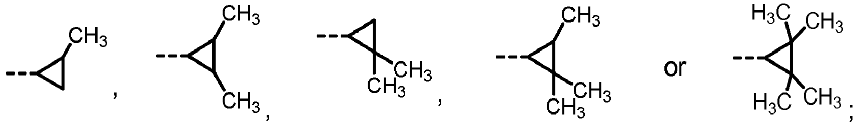 **R³** and **R⁵** represent independently of each other -H, -F, -CI, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and
**R¹⁰** and **R¹¹** have the meanings as defined herein;
or
Ar represents 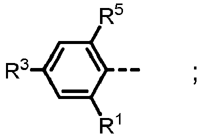 **R¹**, **R³** and **R⁵** represent independently of each other -H, -F, -Cl, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and **R¹⁰** and **R¹¹** have the meanings as defined herein.

Also preferred is a compound of general formula (I), wherein Ar represents 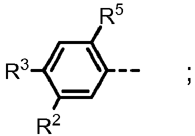 **R²** represents -C(CH₃)₃, -C(CH₃)₂CH₂CH₃ or 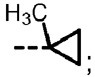 **R³** represents -H or -Cl and **R⁵** represents -OH;
or
Ar represents 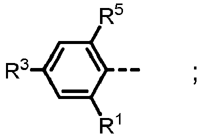 **R¹**, **R³** and **R⁵** represent independently of each other -H, -CH₃, -CH₂CH₃, -CH(CH₃)₂ or -CH₂CH₂CH₃.

Preferred is further a compound of general formula (**IIa**) 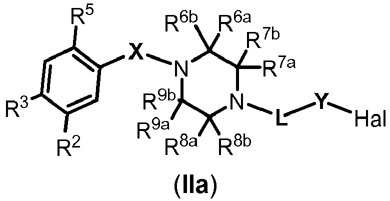 wherein **R²** represents -C(CH₃)R¹⁰R¹¹, -*cyclo*-C₃H₅, *-cyclo-*C₄H₇*,* -*cyclo*-C₅H₉, 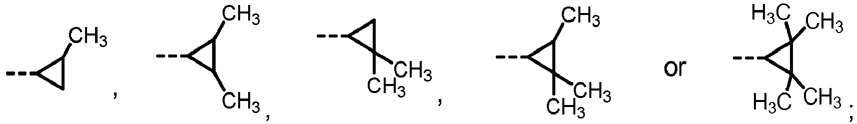 **R³** and **R⁵** represent independently of each other -H, -F, -Cl, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and
**R^{6a}** - **R^{9a}** and **R^{6b}** - **R^{9b}**, **R¹⁰**, **R¹¹ L**, **Y** and **Hal** have the meanings as defined herein.

Further preferred is a compound of general formula (**IIa**), wherein
**R²** represents -C(CH₃)₃, -C(CH₃)₂CH₂CH₃ or 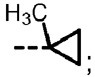 **R³** represents -H or -Cl and **R⁵** represents -OH.

Preferred is further a compound of general formula (**IIb**) 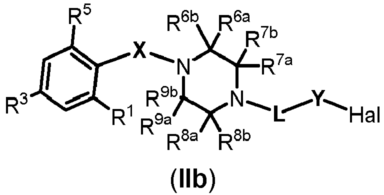 **R¹**, **R³** and **R⁵** represent independently of each other -H, -F, -Cl, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and
**R^{6a}** - **R^{9a}** and **R^{6b}** - **R^{9b}**, **R¹⁰**, **R¹¹ L**, **Y** and **Hal** have the meanings as defined herein.

Further preferred is a compound of general formula (**IIb**), wherein **R¹**, **R³** and **R⁵** represent independently of each other -H, -CH₃, -CH₂CH₃, -CH(CH₃)₂ or -CH₂CH₂CH₃.

A preferred embodiment is directed to a compound of general formula (**IIa**) or (**IIb**), wherein **X** represents -NH-CH₂-CO- . Also preferred is a compound of general formula (**IIa**) or (**IIb**), wherein **Hal** represents -Cl. Preferred is a compound of general formula (**IIa**) or (**IIb**), wherein **R^{6a}** - **R^{9a}** and **R^{6b}** - **R^{9b}** represent -H. Particularly preferred is a compound of general formula (**IIa**) or (**IIb**), when **X** represents -NH-CH₂-CO-, **Hal** represents -Cl and wherein **R^{6a}** - **R^{9a}** and **R^{6b}** - **R^{9b}** represent -H.

More preferred are compounds of formula **(I),** (**IIa**), and (**IIb**), wherein **R^{6a}** - **R^{9a}** and **R^{6b}** - **R^{9b}** represent hydrogen.

Preferred is also a compound of general formula (**IIIa**), (**IIIb**) or (**IIIc**) 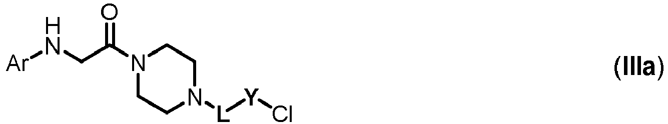 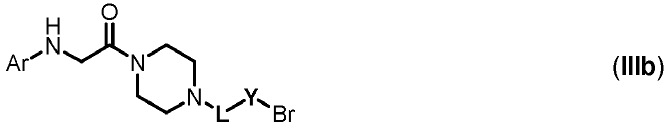 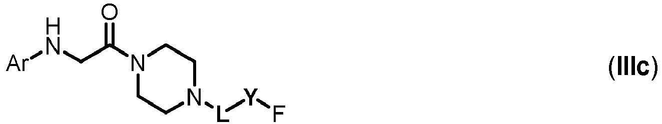 wherein **Ar**, **L and Y** have the meanings as defined herein.

Further preferred is a compound of general formula (**IIIa**), (**IIIb**) or (**IIIc**), wherein
Ar represents 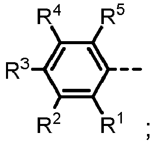 **R¹** - **R⁵** represent independently of each other -H, -F, -Cl, -CN, -CF₃, -OCF₃, -CH₃, -C₂H₅, -C₃H₇, -C₄H₉, -CH(CH₃)₂, -CH₂CH(CH₃)₂, -CH(CH₃)-C₂H₅, -C(CH₃)₃, -C(CH₃)R¹⁰R¹¹, -*cyclo*-C₃H₅, *-cyclo-*C₄H₇, -*cyclo*-C₅H₉, -CH₂-*cyclo*-C₃H₅, -Ph, -OH, 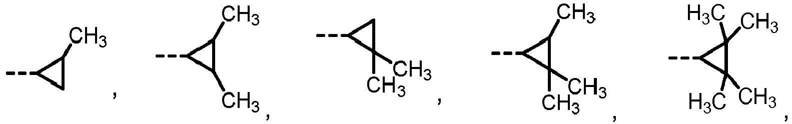 -OCH₃, -OC₂H₅, -OC₃H₇, -OC₄H₉, -OCH(CH₃)₂, -OCH₂CH(CH₃)₂, -OCH(CH₃)-C₂H₅, -OC(CH₃)₃, -O-*cyclo*-C₃H₅, -OCH₂-*cyclo*-C₃H₅, -NH₂, -NH(CH₃), -NH(C₂H₅), -N(CH₃)₂, -N(C₂H₅)₂ or -N(C₃H₇)₂,
**R¹⁰** and **R¹¹** represent independently of each other -CH₃, -CH₂CH₃, -CH₂CH₂CH₃, -CH(CH₃)₂, or **R¹⁰** and **R¹¹** together with carbon atom to which they are attached form a cyclopropane, cyclobutane or cyclopentane ring;
**L** represents -C(O)- or -SO₂- ;
**X** represents -NH-CH₂-CO- or -O-CH₂-CO- ;
**Y** represents 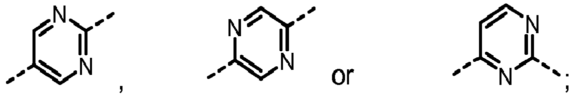 **Hal** represents -Cl, -Br or -F;
or pharmaceutically acceptable salts thereof.

Also preferred is a compound of general formula (**IIIa**), (**IIIb**) or (**IIIc**), wherein **X** represents -NH-CH₂-CO- .

Preferred is moreover a compound of general formula (**IIIa**), (**IIIb**) or (**IIIc**), wherein
Ar represents 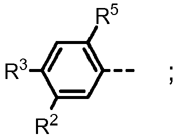 **R²** represents -C(CH₃)R¹⁰R¹¹, -*cyclo*-C₃H₅, *-cyclo-*C₄H₇, -*cyclo*-C₅H₉, 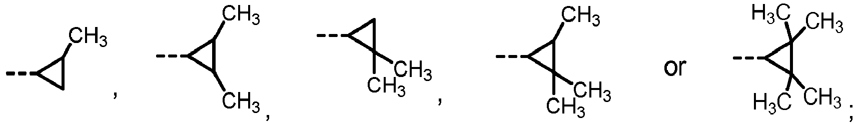 **R³** and **R⁵** represent independently of each other -H, -F, -Cl, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and
**R¹⁰** and **R¹¹** have the meanings as defined herein;
**or**
Ar represents 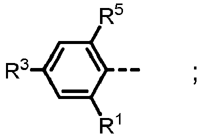 **R¹**, **R³**, and **R⁵** represent independently of each other -H, -F, -Cl, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and **R¹⁰** and **R¹¹** have the meanings as defined herein.

Also preferred is a compound of general formula (**IIIa**), (**IIIb**) or (**IIIc**), wherein
Ar represents 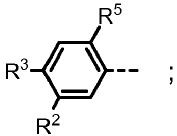 **R²** represents -C(CH₃)₃, -C(CH₃)₂CH₂CH₃ or 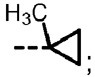 **R³** represents -H or -Cl and **R⁵** represents -OH;
**or**
Ar represents 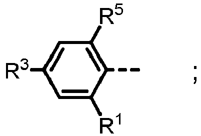 **R¹**, **R³** and **R⁵** represent independently of each other -H, -CH₃, -CH₂CH₃, -CH(CH₃)₂ or -CH₂CH₂CH₃.

Most preferred, the compound of the present invention is selected from the group consisting of:

| | |
|---|---|
| compound **1** | 2-((5-(*tert*-butyl)-2-hydroxyphenyl)amino)-1-(4-(2-chloropyrimidine-5-carbonyl)piperazin-1-yl)ethan-1-one; |
| compound **2** | 1-(4-(2-chloropyrimidine-5-carbonyl)piperazin-1-yl)-2-((2-hydroxy-5-(*tert*-pentyl)phenyl)amino)ethan-1-one; |
| compound **3** | 2-((5-(*tert*-butyl)-4-chloro-2-hydroxyphenyl)amino)-1-(4-(2-chloropyrimidine-5-carbonyl)piperazin-1-yl)ethan-1-one; |
| compound **4** | 2-((4-chloro-2-hydroxy-5-(*tert*-pentyl)phenyl)amino)-1-(4-(2-chloropyrimidine-5-carbonyl)piperazin-1-yl)ethan-1-one; |
| compound **5** | 2-((4-chloro-2-hydroxy-5-(1-methylcyclopropyl)phenyl)amino)-1-(4-(2-chloropyrimidine-5-carbonyl)piperazin-1-yl)ethan-1-one; |
| compound **6** | 2-((4-chloro-2-hydroxy-5-(*tert*-pentyl)phenyl)amino)-1-(4-(2-chloropyrimidine-4-carbonyl)piperazin-1-yl)ethan-1-one; |
| compound **7** | 2-((4-chloro-2-hydroxy-5-(*tert*-pentyl)phenyl)amino)-1-(4-((2-chloropyrimidin-5-yl)sulfonyl)piperazin-1-yl)ethan-1-one; |
| compound **8** | 2-((5-(*tert*-butyl)-4-chloro-2-hydroxyphenyl)amino)-1-(4-(5-chloropyrazine-2-carbonyl)piperazin-1-yl)ethan-1-one; |
| compound **9** | 1-(4-(2-chloropyrimidine-5-carbonyl)piperazin-1-yl)-2-(mesitylamino)ethan-1-one. |

### Chemical synthesis

The inventive 2-halopyrimidines and 5-halopyrazines of general formula (**I**) can be prepared by methods known to one skilled in the art. A retrosynthetic analysis of the inventive compounds of general formula (**I**) is depicted in **Scheme 1**. Suitable starting compounds are properly substituted aniline **4*** and Cbz-protected bromoacetyl piperazine **5***, which are reacted to compound **2*** in the presence of potassium carbonate and DMF as solvent. The inventive 2-halopyrimidines and 5-halopyrazines of general formula (**I**) are obtained by amide or sulfonamide formation between compound **2*** and acyl or sulfonyl chloride **3*** (see **Scheme 2**).

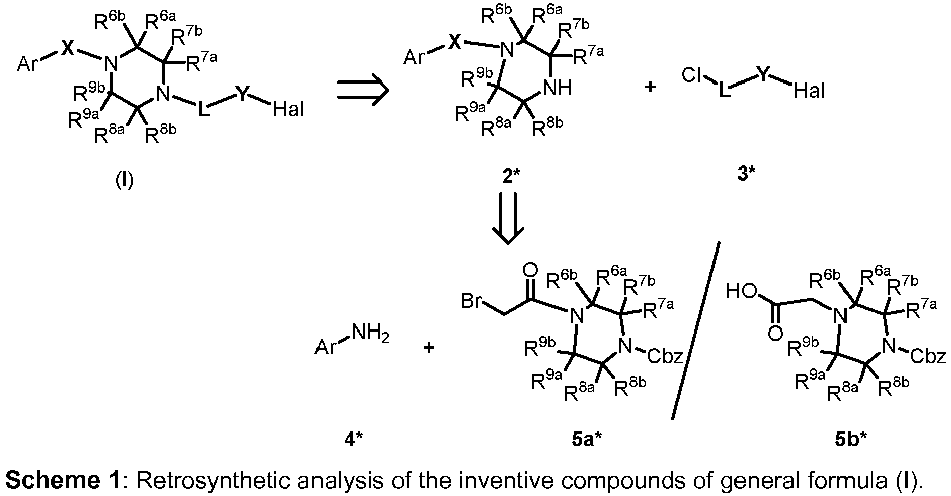

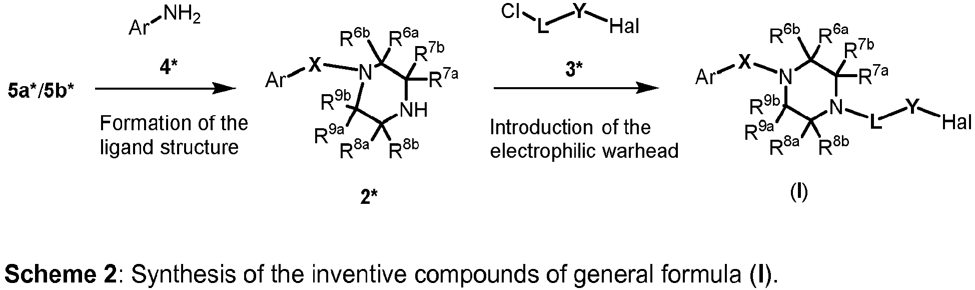

The synthesis of substituted aniline **4*** is exemplarily shown on 4-chloro-2-hydroxy-5-alkyl aniline (see **Scheme 3**). The synthesis commences with the nitration of commercially available para-substituted phenol (**6***) and subsequent protection of the phenol group of compound **7*** with methoxymethyl chloride. Hydrogenation of nitrobenzene **8*** results in an amine **9***, which is afterwards converted to acetamide **10***. Regioselective chlorination was achieved in high yields by using NCS in methylene chloride, followed by hydrolysis of the acetamide to amine **12***. Subsequent alkylation with 1-benzyloxycarbonyl piperazine-4-(2-bromoacetate) (**13***) gave access to piperazine **14***, which was afterwards hydrogenated for removal of the Cbz group. Amidation of compound **15*** with 2-halopyrimidine or 5-halopyrazine acyl or sulfonyl chloride (**3***) results in formation of compound **16***, which was finally converted to 2-halopyrimidine or 5-halopyrazine of formula **17*** by treatment with hydrochloric acid.

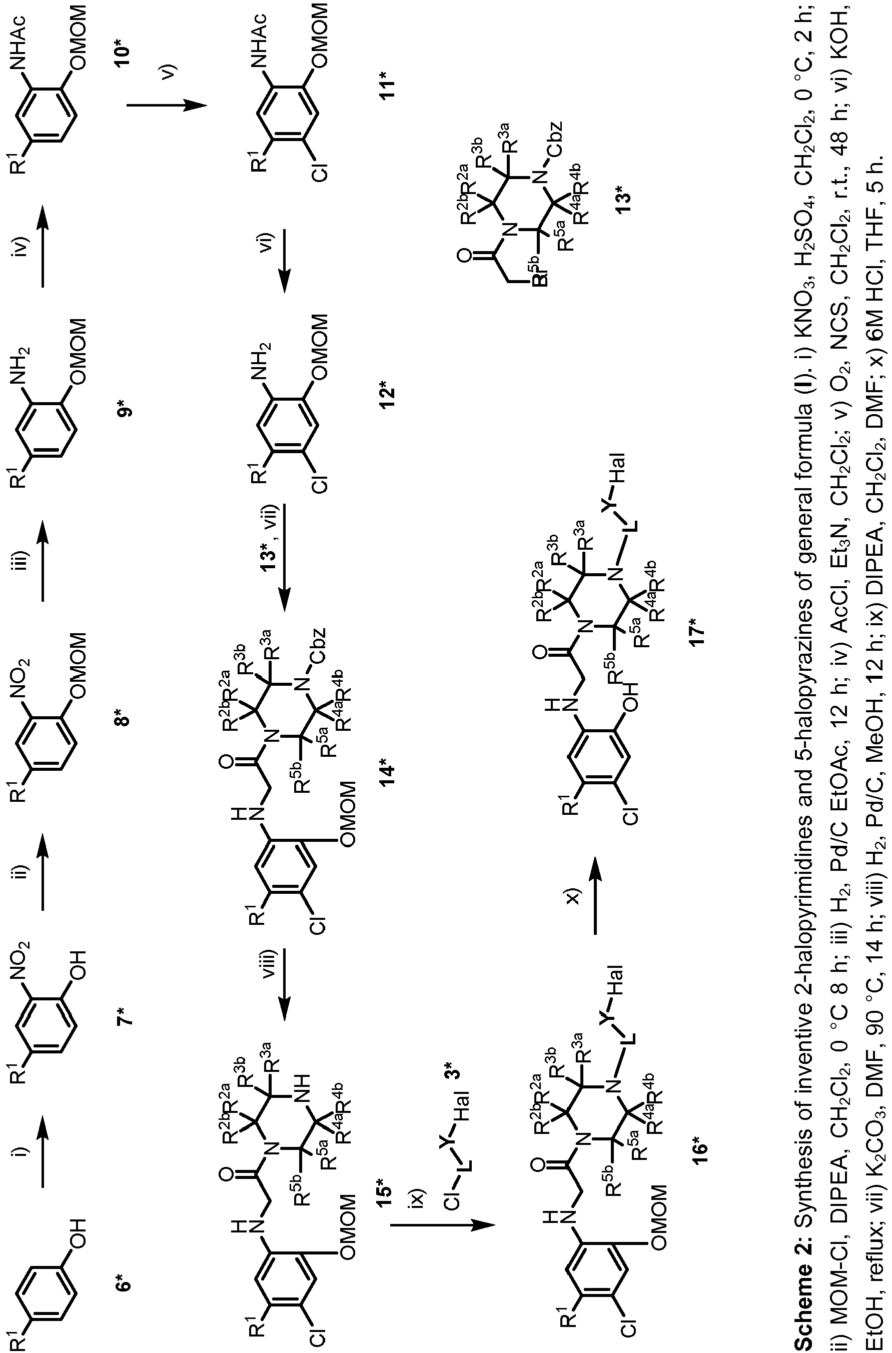

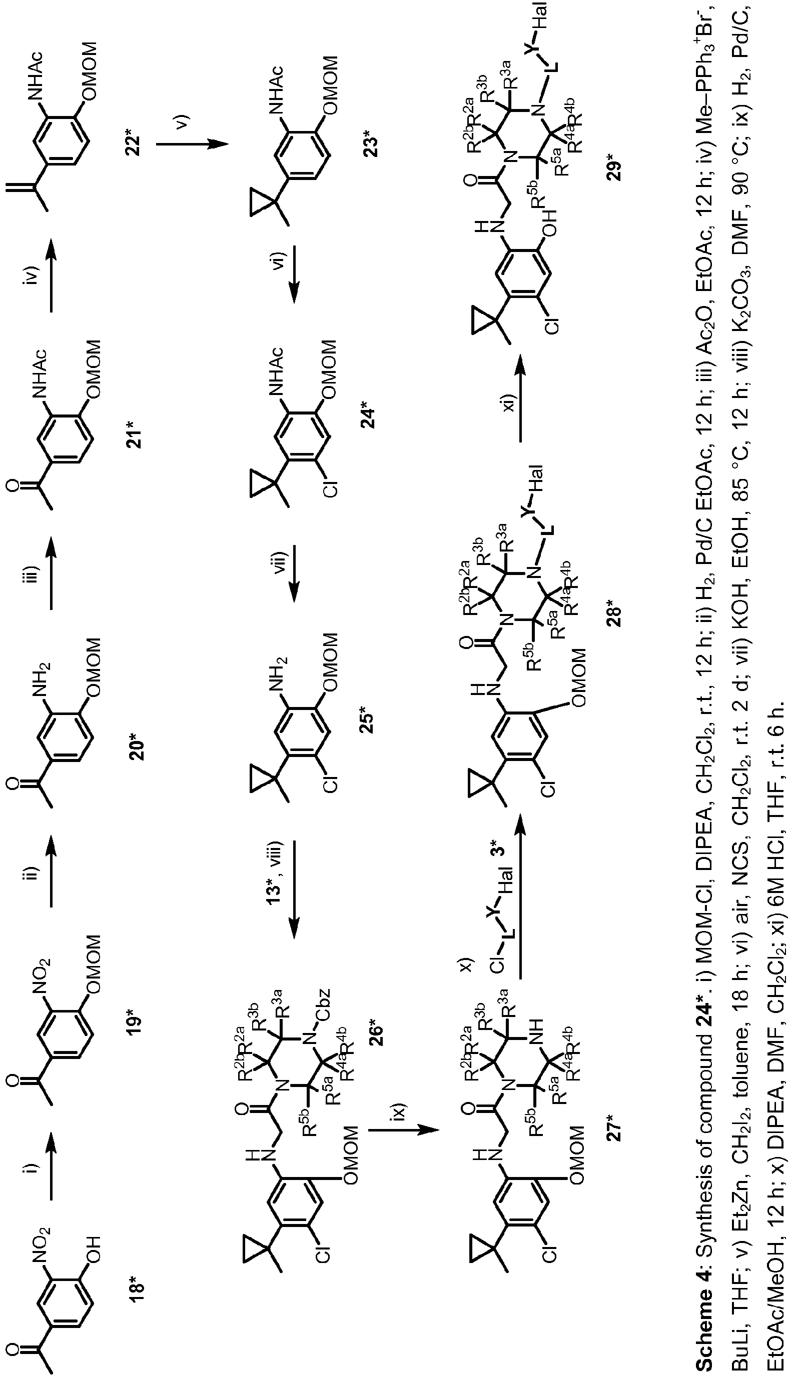

Cyclopropane-substituted 2-halopyrimidines and 5-halopyrazines of the present invention can also be obtained by the synthesis shown in **Scheme 4**. To this extent, substituted acetophenone **18*** is converted to olefin **22*** in 4 steps, including protection of the phenol group with methoxymethyl chloride, hydrogenation to an amine, subsequent reaction to acetamide and Wittig olefination. Afterwards, the cyclopropane ring is installed via Simmons-Smith reaction in the presence of methylene iodide and zinc. Subsequent chlorination and hydrolysis of the acetamide group gave access to compound **25***, which was alkylated with bromoacetate **13*** and afterwards hydrogenated for removal of the Cbz group. Amidation of compound **27*** with 2-halopyrimidine or 5-halopyrazine acyl or sulfonyl chloride (**3***) resulted in formation of compound **28***, which was finally converted to compound **29*** by treatment with hydrochloric acid in tetrahydrofuran.

Some of the compounds of the present invention may be crystallized or recrystallized from solvents such as aqueous and organic solvents. In such cases solvates may be formed. This invention includes within its scope stoichiometric solvates including hydrates as well as compounds containing variable amounts of water that may be produced by processes such as lyophilization.

The compounds of the general formulae (**I**), (**IIa**), (**IIb**), (**IIIa**), (**IIIb**) and (**IIIc**) may exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses.

The compounds of the general formulae (**I**), (**IIa**), (**IIb**), (**IIIa**), (**IIIb**) and (**IIIc**) may form salts with organic or inorganic acids. Examples of suitable acids for such acid addition salt formation are trifluoroacetic acid, hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

### Biological activity

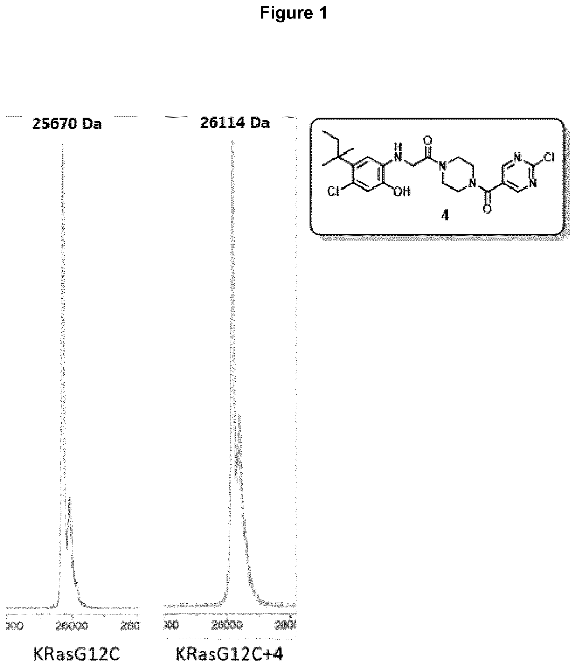

The inventors have surprisingly found that the compounds of the present invention are capable of selectively binding to the KRas G12C mutant by forming an irreversible covalent bond with Cys12, which resides in the switch II pocket. The binding of the inventive compounds to KRas G12C depends on the substitution pattern and the type of the electrophilic warhead represented by **-Y-Hal** moiety, as shown by MALDI and LC-MS (see **Figure 1**).

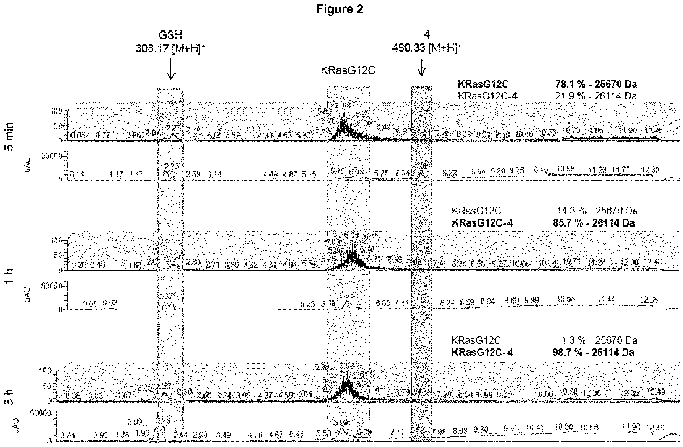

In competitive experiments it was shown that the inventive compounds bind selectively to the KRas G12C mutant in the presence of glutathione (GSH). Thus, a nearly complete covalent adduct formation between compound **4** KRas G12C and was observed in the presence of glutathione by LC-MS (**Figure 2**).

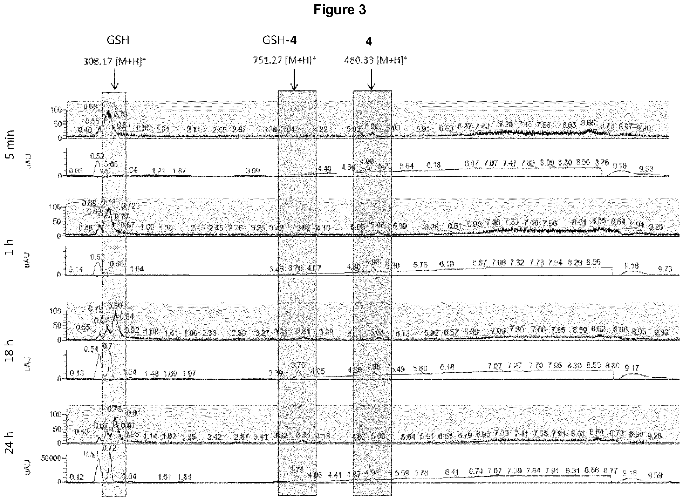

It was also shown that the compounds of the present invention are stable towards hydrolysis under biological conditions, i.e. at 37 °C and physiological pH (7.5) for a prolonged time. LC-MS experiments shown in **Figure 3** revealed that the inventive compound **4** remains unreacted after more than 18 hours incubation at 37 °C in PBS buffer in the presence of glutathione, although glutathione -adduct formation occurs slowly.

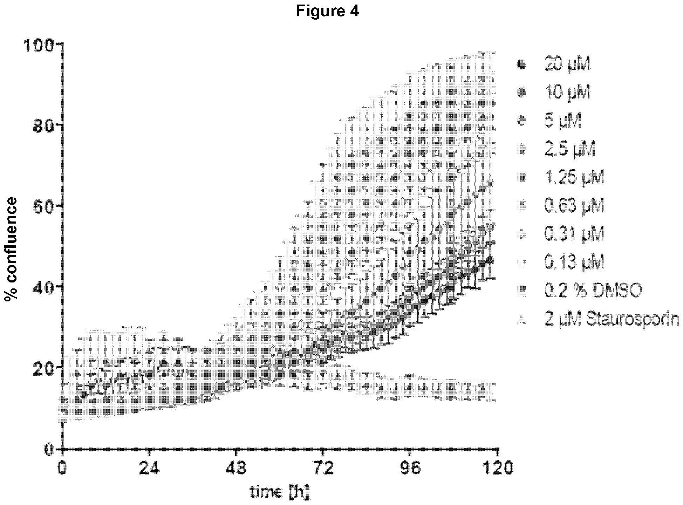

Moreover, the inventors could show in cell proliferation experiments of KRas dependent NCI-H358 cell lines that the compounds of the present invention are able to inhibit the proliferation of cancer cells in a dose dependent manner and are therefore useful for the treatment and/or prophylaxis of proliferative diseases (see **Figure 4**).

Due to the binding of the inventive compounds to Cys12 the switch II of the KRas G12C mutant is locked into an inactive stage, which may be a state distinct from the GTP or GDP bound states of KRas. Further, the altered conformation of the switch II pocket reduces interactions of KRas with effector and regulatory proteins, thereby leading to an interruption of the KRas downstream signaling and to the inhibition of effective proliferation of lung cancer cells, especially non-small cell lung cancer.

### Indications and Pharmaceutical compositions

Therefore, another aspect of the present invention relates to the use of the inventive substituted 2-halopyrimidines and 5-halopyrazines of the formula (I) as drugs, i.e. as pharmaceutically active agents applicable in medicine. 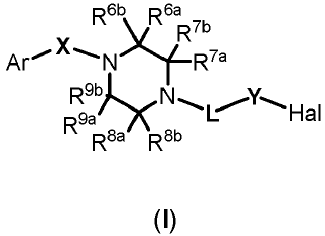 wherein Ar represents 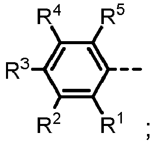 **R¹** - **R⁵** represent independently of each other -H, -F, -Cl, -CN, -CF₃, -OCF₃, -CH₃, -C₂H₅, -C₃H₇, -C₄H₉, -CH(CH₃)₂, -CH₂CH(CH₃)₂, -CH(CH₃)-C₂H₅, -C(CH₃)₃, -C(CH₃)R¹⁰R¹¹, -*cyclo*-C₃H₅, *-cyclo-*C₄H₇, -CH₂-*cyclo*-C₃H₅, -Ph, -OH, 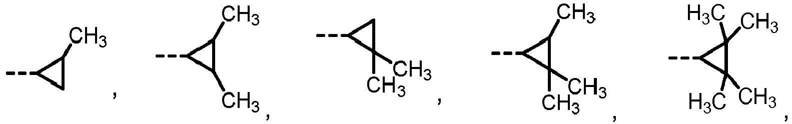 -OCH₃, -OC₂H₅, -OC₃H₇, -OC₄H₉, -OCH(CH₃)₂, -OCH₂CH(CH₃)₂, -OCH(CH₃)-C₂H₅, -OC(CH₃)₃, -O-*cyclo*-C₃H₅, -OCH₂-*cyclo*-C₃H₅, -NH₂, -NH(CH₃), -NH(C₂H₅), -N(CH₃)₂ or -N(C₂H₅)₂;
**R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -O-cyclo-C₃H₅, -OCH₂-cyclo-C₃H₅, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -NHCOCH₃, -NHCOCF₃, -CONH₂, -CONHCH₃, -CONHC₂H₅, -CON(CH₃)₂, -CON(C₂H₅)₂, -SO₂NH₂, -SO₂NHCH₃, -SO₂NHC₂H₅, -SO₂N(CH₃)₂, -SO₂N(C₂H₅)₂, -NHSO₂CH₃, -NHSO₂CF₃ or -NHSO₂C₂H₅; or
**R^{6a}** and **R^{7a}** join to form a 5-membered or 6-membered carbocyclic or heterocyclic ring, or **R^{6b}** and **R^{7b}** join to form a 5-membered or 6-membered carbocyclic or heterocyclic ring, or **R^{8a}** and **R^{9a}** join to form a 5-membered or 6-membered carbocyclic or heterocyclic ring, or **R^{8b}** and **R^{9b}** join to form a 5-membered or 6-membered carbocyclic or heterocyclic ring;
**R¹⁰** and **R¹¹** represent independently of each other -CH₃, -CH₂CH₃, -CH₂CH₂CH₃, -CH(CH₃)₂, or **R¹⁰** and **R¹¹** together with carbon atom to which they are attached form a cyclopropane, cyclobutane or cyclopentane ring;
**L** represents -C(O)- or -SO₂- ;
**X** represents -NH-CH₂-CO- or -O-CH₂-CO- ;
**Y** represents 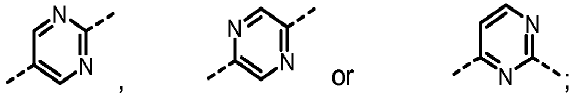 **Hal** represents -Cl, -Br or -F;
or pharmaceutically acceptable salts thereof.

Preferably, **R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -CONH₂, -CONHCH₃, -CONHC₂H₅, -CON(CH₃)₂ or -CON(C₂H₅)₂. Thus, the present invention is also directed to a compound of general formula (**I**), wherein **R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -CONH₂, -CONHCH₃, -CONHC₂H₅, -CON(CH₃)₂ or -CON(C₂H₅)₂.

Preferably, **R^{6a}** and **R^{7a}** join to form a 5-membered or 6-membered carbocyclic or heterocyclic ring, or **R^{6b}** and **R^{7b}** join to form a 5-membered or 6-membered carbocyclic or heterocyclic ring, or **R^{8a}** and **R^{9a}** join to form a 5-membered or 6-membered carbocyclic or heterocyclic ring, or **R^{8b}** and **R^{9b}** join to form a 5-membered or 6-membered carbocyclic or heterocyclic ring, wherein the 5-membered or 6-membered carbocyclic or heterocyclic ring formed between **R^{6a}** and **R^{7a}**, **R^{6b}** and **R^{7b}**, **R^{8a}** and **R^{9a}** and **R^{8b}** and **R^{9b}** is independently selected from: 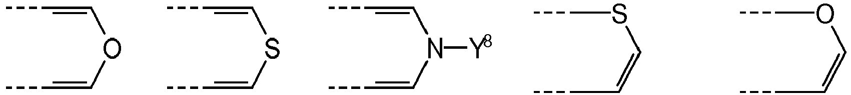 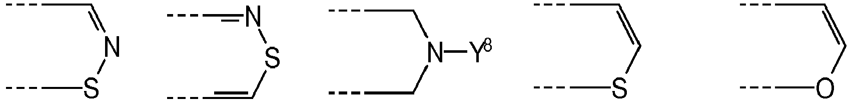 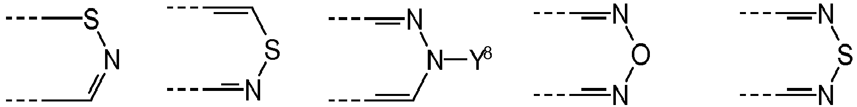 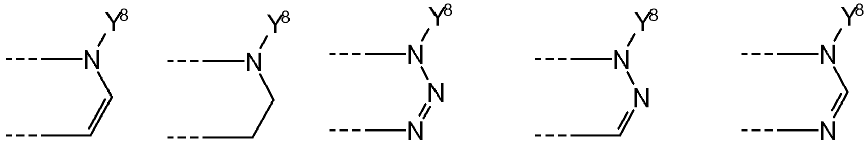 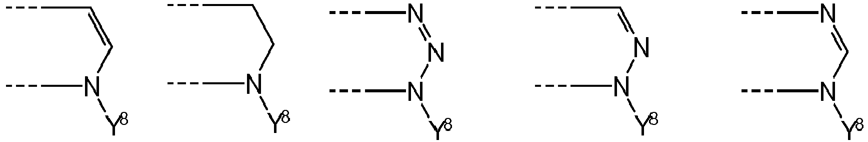 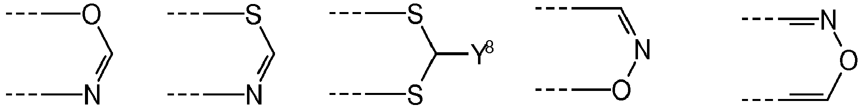 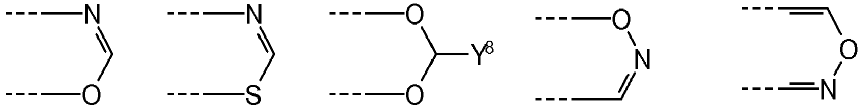 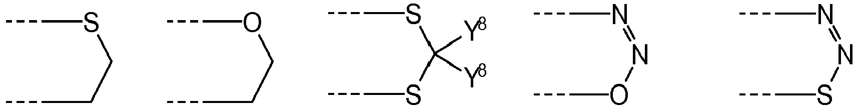 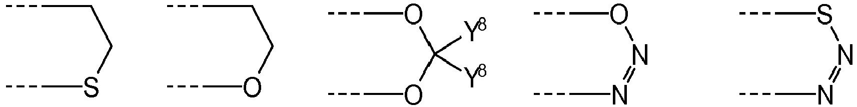 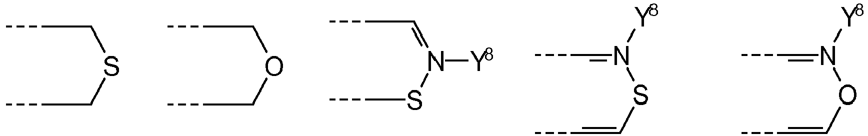 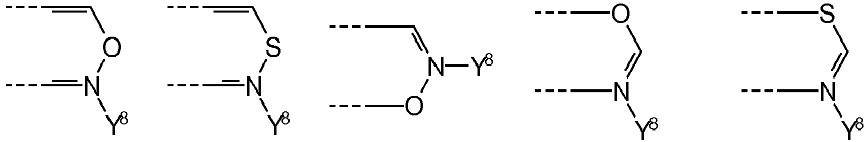 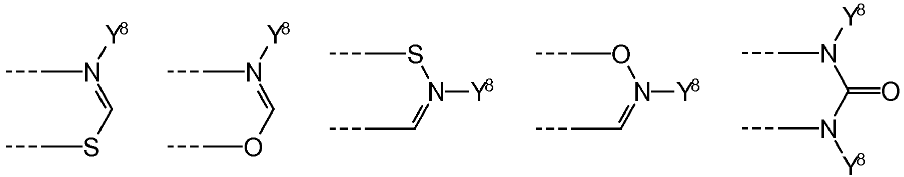 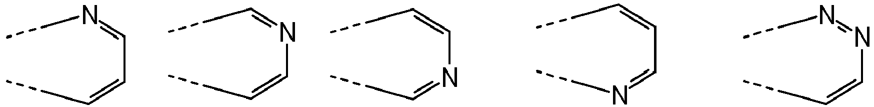 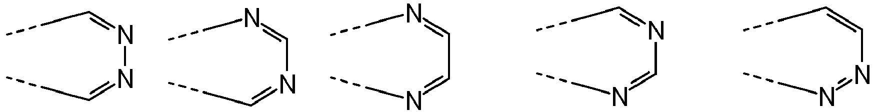 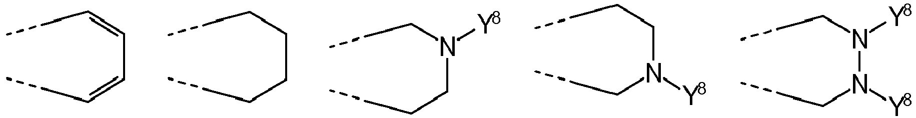 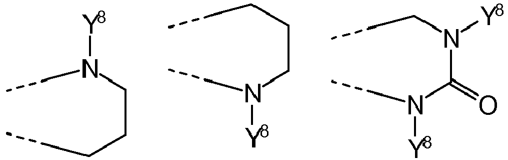 wherein Y⁸ represents -H, -CH₃, -C₂H₅, -C₃H₇, -C₄H₉, -CH(CH₃)₂, -CH₂CH(CH₃)₂, -CH(CH₃)-C₂H₅, -C(CH₃)₃ or -cyclo-C₃H₅.

More preferably, **R^{6a}** - **R^{9a}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -CONH₂, -CONHCH₃, -CONHC₂H₅, -CON(CH₃)₂ or -CON(C₂H₅)₂; and **R^{6b}** - **R^{9b}** represent -H.

Most preferably **R^{6a}** - **R^{9a}** and **R^{6b}** - **R^{9b}** represent -H. Thus, the present invention is also directed to a compound of general formula (**I**), wherein **R^{6a}** - **R^{9a}** and **R^{6b}** - **R^{9b}** represent -H.

In a preferred embodiment, **X** represents -NH-CH₂-CO- . In a further preferred embodiment, **Hal** represents -Cl. Particularly preferred is, when **X** represents -NH-CH₂-CO- and **Hal** represents -Cl.

Surprisingly, it was found that the above-mentioned compounds of the formulae (**I**), (**IIa**), (**IIb**), (**IIIa**), (**IIIb**) and (**IIIc**) as well as the pharmaceutical compositions comprising at least one of said compounds of formulae (**I**), (**IIa**), (**IIb**), (**IIIa**), (**IIIb**) and (**IIIc**) are useful for treatment or prophylaxis of cancer, tumors and proliferative diseases caused by and/or associated with the activating KRas G12C mutation.

The term "mutation", as used herein, means a difference in the amino acid or nucleic acid sequence of a particular protein or nucleic acid (gene, RNA) relative to the wild-type protein or nucleic acid, respectively. A mutated protein or nucleic acid can be expressed from or found on one allele (heterozygous) or both alleles (homozygous) of a gene, and may be somatic or germ line. In the instant invention, mutations are generally somatic. Mutations include sequence rearrangements such as insertions, deletions, and point mutations.

The term "KRas G12C mutation" as used herein refers to a constitutive active form of the KRAS protein caused by a point mutation G12C.

Thus, the compounds of the present invention can be used for prophylaxis and/or treatment of cancers, tumors and proliferative diseases or for the preparation of a pharmaceutical formulation for prophylaxis and/or treatment of cancers, tumors and proliferative diseases, preferably cancer, tumors and proliferative diseases caused by and/or associated with the activating KRas G12C mutation.

As already mentioned, KRas is the most frequently mutated oncogene is tumors. Cancer cell lines harboring KRas mutations have been classified based on KRas dependency for cell viability into KRas dependent and KRas independent groups (Cancer Cell 2009, 15, 489). Examples of KRas dependent cell lines include, but are not restricted to: PANC-1, Mia PaCa-2, Panc-Tu-I, BxPC-3. The compounds of general formula (**I**) are able to inhibit the proliferation of KRas dependent cells, leading to cell death.

More specifically, the cancers, tumors and proliferative diseases that can be treated and/or prevented by the inventive compounds are selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Preferably, the compounds of formulae (**I**), (**IIa**), (**IIb**), (**IIIa**), (**IIIb**) and (**IIIc**) as well as the pharmaceutical compositions comprising at least one of said compounds of formulae (**I**), (**IIa**), (**IIb**), (**IIIa**), (**IIIb**) and (**IIIc**) are useful for treatment or prophylaxis of small-cell lung cancer, non-small cell lung cancer, adenocarcinomas, squamous cell carcinomas, large cell carcinomas and bronchialcarcinoids.

Cancer cells often depend on the survival signaling emanating from oncogene products, particularly from oncogenic KRas, for their survival. The induction of programmed cell death by the loss of such survival signaling, as disclosed for the compounds of the present invention, is especially useful in the treatment of cancer by inducing the death of oncogenic Ras dependent malignant cells. Since all kinds of cancer cells are destroyable through the induction of programmed cell death, all different kinds of cancer and abnormal proliferating cells can be treated with the compounds of the present invention.

In one embodiment, the present invention is directed to a pharmaceutical composition comprising at least one compound of the present invention as active ingredient, together with at least one pharmaceutically acceptable carrier, excipient and/or diluent.

Said pharmaceutical compositions can be used for prophylaxis and/or treatment of cancers, tumors and proliferative diseases or for the preparation of a pharmaceutical formulation for prophylaxis and/or treatment of cancers, tumors and proliferative diseases, preferably cancer, tumors and proliferative diseases caused by and/or associated with the activating KRas G12C mutation.

The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes use of a compound of the formula (**I**) for the preparation of a pharmaceutical formulation for use in the prophylaxis, and/or treatment of cancer, tumors and proliferative diseases.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention.

The pharmaceutical compositions according to the present invention containing at least one compound according to the present invention as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the inventive benzene disulfonamde of general formula (**I**) as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimize the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood, which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilized in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses, such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances, which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Another aspect of this invention provides a method of treating a disease or a medical condition in a patient comprising administering to said patient one or more compound(s) of general formula (**I**) in an amount effective to treat or prevent said disease or condition.

In a particular embodiment, the invention provides a method of treating or preventing of a proliferative disease, a tumor and/or a cancer in a patient, which comprises administering to said patient a therapeutically effective amount of at least one compound of general formula (**I**).

The term "effective amount" means an amount of compound that, when administered to a patient in need of such treatment, is sufficient to
(i) treat or prevent a particular disease, condition, or disorder;
(ii) attenuate, ameliorate, or eliminate one or more symptoms of the particular disease, condition, or disorder; or
(iii) prevent or delay the onset of one or more symptoms of the particular disease, condition, or disorder described herein.

The amount of a compound of general formula (**I**) that will correspond to such an amount will vary depending upon factors such as the particular compound, disease condition and its severity, the identity (e.g., weight) of the patient in need of treatment, but can nevertheless be routinely determined by one skilled in the art.

### Description of the Figures

**Figure 1** shows MALDI-TOF mass spectra (positive ion mode) of KRas G12C before (left) and after 30 minutes incubation time with compound **4** (2.4 µM, 1.2 eq.).
**Figure 2** shows LC-MS traces of the KRas G12C, compound **4** and glutathione after 5 minutes (top), 1 hour (center) and 5 hours (bottom). Nearly complete covalent adduct formation was observed after 1 hour under these experimental conditions. No glutathione-**4** adduct formation could be observed up to 5 hours.
**Figure 3** shows LC-MS traces of a mixture of compound **4** and glutathione after 5 minutes, 1 hour, 18 hours and 24 hours incubation in PBS buffer at 37 °C. Compound **4** showed a good stability under these conditions. Formation of the glutathione-**4** covalent adduct occurs slowly and ca. 50% of compound **4** remains unreacted after 18 hours incubation time.
**Figure 4** shows cell proliferation by means of live cell imaging of KRas dependent NCI-H358 cell lines treated with different doses of compound **4**. Cell indices ±s.d. were measured in technical triplicates and biological duplicates. Staurosporin was used as control compound.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### A. Chemical Synthesis

### General Information:

All reactions involving air- or moisture-sensitive reagents or intermediates were carried out in flame dried glassware under an argon atmosphere. Dry solvents (THF, toluene, MeOH, DMF) were used as commercially available; CH₂Cl₂ was purified by the Solvent Purification System M-BRAUN Glovebox Technology SPS-800. Analytical thin-layer chromatography (TLC) was performed on Merck silica gel aluminium plates with F-254 indicator. Compounds were visualized by irradiation with UV light or potassium permanganate staining. Column chromatography was performed using silica gel Merck 60 (particle size 0.040-0.063 mm). ¹H-NMR and ¹³C-NMR were recorded on a Bruker DRX400 (400 MHz), Bruker DRX500 (500 MHz), INOVA500 (500 MHz) and DRX600 (600 MHz) at 300 K using CDCl₃, CD₃OD or (CD₃)₂SO as solvents. All resonances are reported relative to TMS. Spectra were calibrated relative to solvent's residual proton and carbon chemical shift: CDCl₃ (δ = 7.26 ppm for ¹H NMR and δ = 77.16 ppm for ¹³C NMR); CD₃OD (δ = 3.31 ppm for ¹H NMR and δ = 49.00 ppm for ¹³C NMR); (CD₃)₂SO: δ= 2.50 ppm for ¹H NMR and δ= 39.52 ppm for ¹³C NMR). Multiplicities are indicated as: br s (broadened singlet), s (singlet), d (doublet), t (triplet), q (quartet), quin (quintet), m (multiplet); and coupling constants (J) are given in Hertz (Hz). High resolution mass spectra were recorded on a LTQ Orbitrap mass spectrometer coupled to an Acceka HPLC-System (HPLC column: Hypersyl GOLD, 50 mm x 1 mm, particle size 1.9 µm, ionization method: electron spray ionization). Atorvastatin was purchased from Sequoia Reseach Products. All other chemicals and solvents were purchased from Sigma-Aldrich, Fluka, TCI, Acros Organics, ABCR and Alfa Aesar. Unless otherwise noted, all commercially available compounds were used as received without further purifications.

### General synthetic routes:

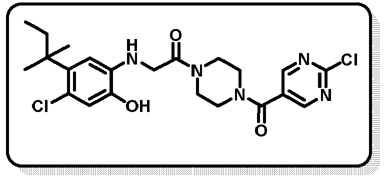

**2-((4-chloro-2-hydroxy-5-(tert-pentyl)phenyl)amino)-1-(4-(2-chloropyrimidine-5-carbonyl)piperazin-1-yl)ethan-1-one:** A solution of 2-chloropyrimidine-5-carboxylic acid (100 mg, 0.63 mmol, 1eq) in tetrahydrofuran (2 mL) was treated with 2 drops of N,N-dimethylformamide. Oxalyl chloride (0.107 mL, 1.26 mmol) was added dropwise over 3 minutes and the mixture was stirred at room temperature for 2 hours. The solution was concentrated and dried under vacuum to give the crude acid chloride. A solution of the acid chloride in DCM (4 mL) was added dropwise to a solution of 2-((4-chloro-2-(methoxymethoxy)-5-(*tert-*pentyl)phenyl)amino)-1-(piperazin-1-yl)ethan-1-one (0.63 mmol) in DCM (6 mL) at -78 °C. Reaction mixture was slowly warmed to room temperature. After stirring at room temperature for 2 hours, the mixture was quenched by adding water and organic layer was extracted with DCM, dried over Na₂SO₄, and purified by silica gel column chromatography to give the title compound.

To the above obtained compound in THF (2 mL) was added HCl (6M, 2 mL) and the mixture was stirred for 1.5 h. Again, HCl (6M, 1 mL) was introduced, stirred for another 1 h. This process was continued until the consumption of starting compound was observed (UHPLC). After completion of the reaction, it was quenched by slowly adding water, sat., NaHCO₃. The product was extracted with EtOAc, dried over Na₂SO₄, filtered over silica gel and concentrated to give the pure product (72% yield for 2 steps). **¹H NMR (400 MHz, DMSO-*d*₆)** δ 9.60 (s, 1H), 8.87 (s, 2H), 6.63 (s, 1H), 6.45 (s, 1H), 3.94 (d, *J* = 10.5 Hz, 2H), 3.77 - 3.46 (m, 8H), 1.83 (q, *J* = 7.4 Hz, 2H), 1.33 (s, 3H), 1.31 (s, 3H), 0.58 (t, *J* = 7.3 Hz, 3H). **¹³C NMR (101 MHz, dmso)** δ 168.67, 164.43, 161.14, 159.43, 143.29, 135.71, 135.22, 129.49, 118.88, 116.78, 111.56, 47.52, 45.22, 44.00, 42.23, 40.83, 40.62, 40.41, 40.20, 39.99, 39.78, 39.58, 39.34, 35.05, 33.15, 31.10, 28.90, 10.03; **HRMS (ESI):** m/z calcd for C₂₂H₂₈O₃N₅Cl₂ [M+H]⁺ : 480.15637, found: 480.15604.

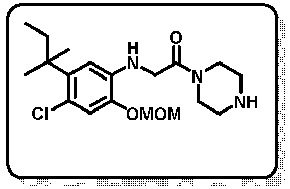

**2-((4-chloro-2-(methoxymethoxy)-5-(*tert*-pentyl)phenyl)amino)-1-(piperazin-1-yl)ethan-1-one:** A solution of benzyl 4-((4-chloro-2-(methoxymethoxy)-5-(*tert-*pentyl)phenyl)glycyl)piperazine-1-carboxylate (3.00 g, 5.79 mmol) in MeOH (30 mL) was added slowly portion wise 10% Pd/C (0.60 g), and it was stirred under H₂ atmosphere for 15 h. The mixture was filtered through a pad of celite, the celite pad was washed with EtOAc. The filtrate was concentrated *in vacuo* to provide the pure product in a quantitative yield. **¹H NMR (400 MHz, Chloroform-*d*)** δ 7.01 (s, 1H), 6.44 (s, 1H), 5.31 (s, 1H), 5.16 (s, 2H), 3.88 (d, *J* = 2.7 Hz, 2H), 3.66 (t, *J* = 5.1 Hz, 2H), 3.49 (s, 3H), 3.45 (dd, *J* = 6.3, 4.3 Hz, 2H), 2.90 (dt, *J* = 17.1, 5.1 Hz, 4H), 1.94 (q, *J* = 7.5 Hz, 2H), 1.83 (s, 1H), 1.40 (s, 6H), 0.66 (t, *J* = 7.4 Hz, 3H). **¹³C NMR (101 MHz, cdcl₃)** δ 167.39, 142.93, 138.37, 136.09, 120.48, 117.43, 110.88, 95.26, 77.32, 77.00, 76.68, 56.22, 46.18, 45.79, 45.57, 44.96, 43.13, 39.25, 32.72, 28.20, 9.42; **HRMS (ESI):** m/z calcd for C₁₉H₃₁O₃N₃Cl [M+H]⁺: 384.20485, found: 384.20605.

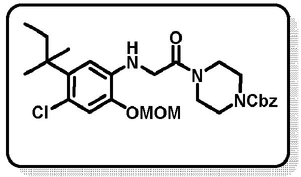

**benzyl 4-((4-chloro-2-(methoxymethoxy)-5-(*tert*-pentyl)phenyl)glycyl)-piperazine-1-carboxylate:** To a solution containing 4-chloro-2-(methoxymethoxy)-5-(*tert*-pentyl)aniline (3.50 g, 13.58 mmol) in DMF was added oven dried K₂CO₃ (3.75 g, 27.16 mmol) at room temperature. The resulted reaction mixture was stirred for 30 min. and to this was added bromide (13.58 mmol) and continued the stirring at 90 °C for 18 h. The residue was poured into water and extracted with ethyl acetate. The organic layer was combined, washed with water and brine, and dried over anhydrous Na₂SO₄. The desired product was obtained after column chromatography (yield 66%). **¹H NMR (400 MHz, Chloroform-*d*)** δ 7.41 - 7.28 (m, 5H), 7.01 (s, 1H), 6.45 (s, 1H), 5.24 (s, 1H), 5.16 (s, 4H), 3.90 (s, 2H), 3.77 - 3.42 (m, 8H), 3.48 (s, 3H), 1.94 (q, *J* = 7.5 Hz, 2H), 1.39 (s, 6H), 0.66 (t, *J* = 7.4 Hz, 3H). **¹³C NMR (101 MHz, cdcl₃)** δ 167.69, 155.02, 142.90, 138.39, 136.20, 135.87, 128.53, 128.22, 128.00, 120.69, 117.43, 110.92, 95.24, 77.32, 77.00, 76.68, 67.54, 56.19, 45.13, 44.16, 43.57, 41.74, 39.23, 32.66, 28.16, 9.39.

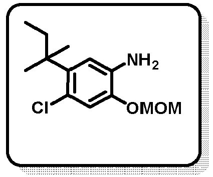

**4-chloro-2-(methoxymethoxy)-5-(*tert*-pentyl)aniline:** To a solution of N-(2-(methoxymethoxy)-5-(*tert*-pentyl)phenyl)acetamide (2.00 g, 8.02 mmol) in DCM (20 mL) at room temperature NCS (8.02 mmol, 1 eq) was added and the resulting reaction mixture was stirred for 12 h. After completion of the reaction (UHPLC) it was concentrated and purified by silica gel column to give quantitative yield of the product.

To the above product in EtOH (10 mL) KOH (4.0 M, 20 mL) was added and the resulting reaction mixture was heated at 90 °C for 15 h. After completion of the reaction it was diluted to pH = 7 and extracted with EtOAc and dried over Na₂SO₄. The desired product was obtained after silica gel column chromatography (90 %). **¹H NMR (400 MHz, Chloroform-*d*)** δ 7.01 (s, 1H), 6.71 (s, 1H), 5.14 (s, 2H), 3.73 (s, 1H), 3.49 (s, 3H), 1.92 (q, *J* = 7.5 Hz, 2H), 1.36 (s, 6H), 0.65 (t, *J* = 7.5 Hz, 3H). **¹³C NMR (101 MHz, cdcl₃)** δ 142.96, 138.65, 134.59, 121.89, 118.09, 116.03, 95.28, 77.32, 77.00, 76.68, 56.17, 39.05, 32.62, 28.15, 9.38; **HRMS (ESI):** m/z calcd for C₁₉H₃₁O₃N₃Cl [M+H]⁺ : 384.20485, found: 384.20605.

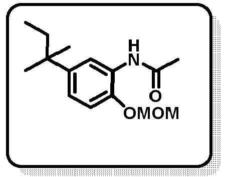

**N-(2-(methoxymethoxy)-5-(*tert*-pentyl)phenyl)acetamide:** To a solution of 2-amino-4-(*tert*-pentyl)phenol (5.0 g, 1.0 equiv) in ethyl acetate (50 mL) at 0 °C, acetic anhydride (2.00 equiv) was added slowly over 15 min. The mixture was allowed to warm to room temperature over 5 h during which a solid started to precipitate out of the solution (If not also). The mixture was concentrated under reduced pressure to leave -10% of the initial ethyl acetate. To this slurry hexanes (60 mL) was added, stirred for 10 min and filtered. The solid residue was washed with hexanes (15 mL) and dried to give the pure product.
To the above obtained product (5 g, 1 eq) in DCM (25 mL) DIPEA (1.5 eq) was added and to this at 0 °C MOMCI (1.2 eq) was added. The resulting reaction mixture was left at rt overnight. After completion of the reaction, it was poured into water (50 mL) and the organic layer was extracted with DCM. The combined organic layers were washed with brine, dried over Na₂SO₄, filtered and concentrated *in vacuo.* The residue was purified by silica gel column chromatography to afford the desired product in quantitative yield. **¹H NMR (400 MHz, Chloroform-*d*)** δ 8.38 (d, *J* = 2.3 Hz, 1H), 7.76 (bs, 1H), 7.03 (d, *J* = 3.2 Hz, 1H), 6.96 (d, *J* = 2.4 Hz, 1H), 5.19 (s, 2H), 3.48 (s, 3H), 2.19 (s, 3H), 1.61 (q, *J* = 7.4 Hz, 2H), 1.24 (s, 6H), 0.68 (d, *J* = 7.4 Hz, 3H).

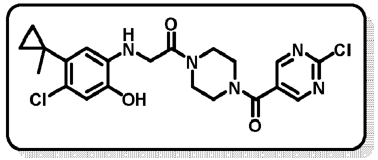

**2-((4-chloro-2-hydroxy-5-(1-methylcyclopropyl)phenyl)amino)-1-(4-(2-chloropyrimidine-5-carbonyl)piperazin-1-yl)ethan-1-one:** A solution of 2-chloropyrimidine-5-carboxylic acid (100 mg, 0.63 mmol, 1eq) in tetrahydrofuran (2 mL) was treated with 2 drops of N,N-dimethylformamide. Oxalyl chloride (0.107 mL, 1.26 mmol) was added dropwise over 3 minutes and the mixture was stirred at room temperature for 2 hours. The solution was concentrated and dried under vacuum to give the crude acid chloride. A solution of the acid chloride in DCM (4 mL) was added dropwise to a solution of 2-((4-chloro-2-(methoxymethoxy)-5-(1-methylcyclopropyl)phenyl)amino)-1-(piperazin-1-yl)ethan-1-one (0.63 mmol) in DCM (6 mL) at -78 °C. The reaction mixture was slowly warmed to room temperature. After stirring at room temperature for 2 hours, the mixture was quenched by adding water. The organic layer was extracted with DCM, dried over Na₂SO₄ and purified by silica gel column chromatography to give the title compound.
To the above compound in THF (2 mL) was added HCl (6 M, 2 mL) and the mixture was stirred for 1.5 h. Again, HCl (6 M, 1 mL) was introduced and the mixture stirred for another 1 h. This process was continued until the consumption of starting compound was observed (UHPLC). After completion of the reaction, it was quenched by slowly adding water and sat. NaHCO₃. The product was extracted with EtOAc, dried over Na₂SO₄, filtered over silica gel and concentrated to give the pure product (70% yield for 2 steps).
**¹H NMR (400 MHz, DMSO-*d₆*)** δ 9.62 (s, 1H), 8.85 (s, 2H), 6.59 (s, 1H), 6.53 - 6.34 (m, 1H), 5.06 (t, *J* = 4.6 Hz, 1H), 3.91 (s, 2H), 3.76 - 3.32 (m, 9H), 3.29 (d, *J* = 2.5 Hz, 6H), 2.45 (p, *J* = 1.9 Hz, 1H), 1.20 (s, 4H), 0.63 (d, *J* = 23.0 Hz, 4H). **¹³C NMR (101 MHz, dmso)** δ 168.56, 164.41, 161.13, 159.42, 143.89, 136.27, 134.54, 129.46, 120.76, 114.40, 113.00, 47.45, 45.14, 44.39, 43.97, 42.52, 42.20, 40.80, 40.59, 40.38, 40.18, 39.97, 39.76, 39.55, 31.07, 25.93, 20.76, 14.80. **HRMS (ESI):** m/z calcd for C₂₂H₂₄O₃N₅Cl₂, [M+H]⁺ : 464.1251, found: 464.1249.

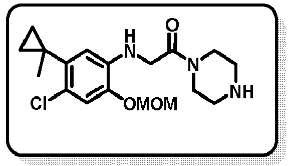

**2-((4-chloro-2-(methoxymethoxy)-5-(1-methylcyclopropyl)phenyl)amino)-1-(piperazin-1-yl)ethan-1-one:** To a solution of benzyl 4-((4-chloro-2-(methoxymethoxy)-5-(1-methylcyclopropyl)phenyl)glycyl)piperazine-1-carboxylate (1.00 g, 1.99 mmol) in MeOH (10 mL) was added slowly portion wise 10% Pd/C (0.20 g), and it was stirred under H₂ atmosphere for 15 h. The mixture was filtered through a pad of celite, the celite pad was washed with EtOAc. The filtrate was concentrated *in vacuo* to provide the pure product in a quantitative yield. **¹H NMR (400 MHz, Chloroform-d)** δ 6.99 (s, 1H), 6.42 (s, 1H), 5.33 (s, 1H), 5.15 (s, 2H), 3.86 (d, *J* = 2.4 Hz, 2H), 3.67 (t, *J* = 3.7 Hz, 2H), 3.47 (s, 6H), 2.91 (dt, *J* = 18.6, 5.1 Hz, 4H), 2.47 (s, 2H), 1.30 (s, 3H), 0.81 - 0.75 (m, 2H), 0.72 - 0.57 (m, 2H). **¹³C NMR (101 MHz, cdcl₃)** δ 167.59, 143.73, 137.66, 136.86, 122.64, 115.55, 112.69, 95.51, 77.57, 77.25, 76.93, 56.43, 46.25, 45.88, 45.57, 45.22, 43.12, 25.50, 20.86, 14.61; **HRMS (ESI):** m/z calcd for C₁₉H₂₆ClN₃O₃, [M+H]⁺ : 367.1663, found: 367.1674.

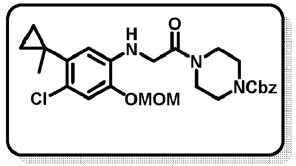

**Benzyl 4-((4-chloro-2-(methoxymethoxy)-5-(1-methylcyclopropyl)phenyl)-glycyl)piperazine-1-carboxylate:** To a solution containing 4-chloro-2-(methoxymethoxy)-5-(1-methylcyclopropyl)aniline (1.00 g, 4.14 mmol), in DMF was added K₂CO₃ (1.14 g, 8.27 mmol) at room temperature. The resulting reaction mixture was stirred for 30 minutes and to this bromide (4.14 mmol)) was added and continued to stir at 90 °C for 18 h. The residue was poured into water and extracted with ethyl acetate. The organic layer was combined, washed with water and brine and dried over anhydrous Na₂SO₄. The desired product was obtained after column chromatography. **¹H NMR (400 MHz, Chloroform-*d*)** δ 7.43 - 7.29 (m, 5H), 7.00 (d, *J* = 0.5 Hz, 1H), 6.43 (s, 1H), 5.20 - 5.08 (m, 4H), 3.89 (s, 2H), 3.77 - 3.49 (m, 8H), 3.48 (d, *J* = 0.6 Hz, 3H), 1.31 (s, 3H), 0.83 - 0.75 (m, 2H), 0.73 - 0.63 (m, 2H). **¹³C NMR (101 MHz, cdcl₃)** δ 167.87, 155.31, 143.75, 137.71, 136.71, 136.48, 128.82, 128.50, 128.28, 122.87, 115.60, 112.75, 95.53, 77.56, 77.44, 77.24, 76.92, 67.83, 56.44, 45.39, 44.42, 43.88, 42.03, 25.50, 20.87, 14.63. **HRMS (ESI):** m/z calcd for C₂₈H₃₃O₅N₃Cl [M+H]⁺ : 502.2103, found: 502.2115.

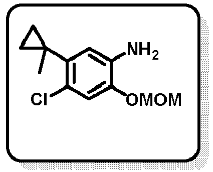

**4-chloro-2-(methoxymethoxy)-5-(1-methylcyclopropyl)aniline:** To a solution of N-(2-(methoxymethoxy)-5-(1-methylcyclopropyl)phenyl)acetamide (2.00 g, 8.02 mmol) in DCM (20 mL) at room temperature was added NCS (8.02 mmol, 1 eq) and the resulting reaction mixture was stirred for 12 h. After completion of the reaction (UHPLC) it was concentrated and purified by silica gel column chromatography to give the title compound in quantitative yield.
To the above obtained product in EtOH (15 mL) was added KOH (4.0 M, 20 mL) and the resulting reaction mixture was heated at 90 °C for 15 h. After completion of the reaction it was diluted to pH = 7, extracted with EtOAc and dried over Na₂SO₄. The desired product was obtained after silica gel column chromatography (yield 90%). **¹H NMR (400 MHz, Chloroform-*d*)** δ 7.00 (s, 1H), 6.71 (s, 1H), 5.14 (s, 2H), 3.73 (s, 2H), 3.49 (s, 3H), 1.30 (s, 3H), 0.79 - 0.74 (m, 2H), 0.70 - 0.65 (m, 2H). **¹³C NMR (101 MHz, cdcl₃)** δ 143.79, 137.98, 135.49, 124.12, 117.90, 116.27, 95.55, 77.55, 77.23, 76.92, 56.40, 25.43, 20.52, 14.44. **HRMS (ESI):** m/z calcd for C1₃H₁₇O₂NCl [M+H]+ : 242.0942, found: 242.0946.

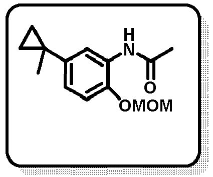

**N-(2-(methoxymethoxy)-5-(1-methylcyclopropyl)phenyl)acetamide:** Diethylzinc (1.0 M in hexanes, 42.50 mL, 42.5 mmol) was added to a mixture of N-(2-(methoxymethoxy)-5-(prop-1-en-2-yl)phenyl)acetamide (1.00 g, 4.25 mmol) in dichloroethane (15 mL) and diiodomethane (3.45 mL, 42.5 mmol) at 0°C, the resulted mixture was stirred at 0 °C for 0.5 h and allowed to warm to rt for 2 h. After completion of the reaction, which was monitored by UHPLC, the opaque mixture was quenched with saturated aqueous NH₄Cl. The aqueous phase was extracted with CH₂Cl₂ and the combined organic layers were washed with saturated aqueous NaHCO₃ and brine. The organic layers were dried over sodium sulfate and the title compound was obtained after silica gel column chromatography (yield 70%). **¹H NMR (400 MHz, Chloroform-d)** δ 8.28 (s, 1H), 7.75 (s, 1H), 7.00 (dd, *J* = 8.5, 1.2 Hz, 1H), 6.89 (dd, *J* = 8.5, 2.2 Hz, 1H), 5.17 (s, 2H), 3.47 (s, 3H), 2.88 (s, 2H), 2.18 (s, 3H), 1.35 (s, 3H), 0.80 (q, *J* = 4.1, 3.6 Hz, 2H), 0.65 (t, *J* = 3.1 Hz, 2H). **HRMS (ESI):** m/z calcd for C₁₅H₂₀O₃N [M+H]⁺ : 250.1438, found: 250.1442.

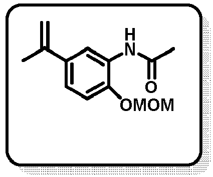

**N-(2-(methoxymethoxy)-5-(prop-1-en-2-yl)phenyl)acetamide:** To a known N-(5-acetyl-2-hydroxyphenyl)acetamide (5 g, 1 eq) in DCM (30 mL) was added DIPEA (1.5 eq) and to this at 0 °C was added MOMCI (1.2 eq). The resulting reaction mixture was left at rt overnight. After completion of the reaction, the mixture was poured into water (50 mL) and the organic layer was extracted with DCM. The combined organic layers were washed with brine and dried over Na₂SO₄. The residue was purified by silica gel column chromatography to afford the desired product in quantitative yield.
Methyltriphenylphosphonium bromide (14.91 g, 41.73 mmol) was suspended in tetrahydrofuran (30 mL), and n-butyllithium (2.5 M in hexane 14.47 mL, 36.16 mmol) was added dropwise under ice-cooling. After stirring at room temperature for 30 minutes, a solution of the above obtained compound (27.82 mmol) in tetrahydrofuran (20 ml) was added under ice-cooling, and the mixture was stirred at room temperature for 5 h. Ethyl acetate and water were added and the mixture was extracted with ethyl acetate. The organic layer was washed with water and saturated sodium chloride solution and dried over anhydrous sodium sulfate. The residue was purified by chromatography (80% yield). **¹H NMR (400 MHz, Chloroform-*d*)** δ 8.53 (d, *J* = 2.2 Hz, 1H), 7.73 (s, 1H), 7.12 (dd, *J* = 8.6, 2.3 Hz, 1H), 7.06 (d, *J* = 8.6 Hz, 1H), 5.32 (dt, *J* = 1.5, 0.8 Hz, 1H), 5.22 (d, *J* = 0.7 Hz, 2H), 5.07 - 4.96 (m, 1H), 3.50 (s, 3H), 2.21 (s, 3H), 2.17 - 2.07 (m, 3H).

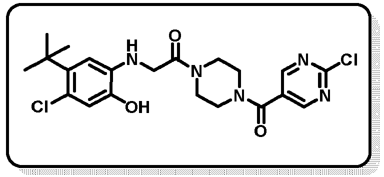

**2-((5-(*tert*-butyl)-4-chloro-2-hydroxyphenyl)amino)-1-(4-(2-chloropyrimidine-5-carbonyl)piperazin-1-yl)ethan-1-one:** A solution of 2-chloropyrimidine-5-carboxylic acid (100 mg, 0.63 mmol, 1eq) in tetrahydrofuran (2 mL) was treated with 2 drops of N,N-dimethylformamide. Oxalyl chloride (0.107 mL, 1.26 mmol) was added dropwise over 3 minutes and the mixture was stirred at room temperature for 2 hours. The solution was concentrated and dried under vacuum to give the crude acid chloride. A solution of the acid chloride in DCM (4 mL) was added dropwise to a solution of 2-((5-(*tert*-butyl)-4-chloro-2-(methoxymethoxy)-phenyl)amino)-1-(piperazin-1-yl)ethan-1-one (0.63 mmol) in DCM (6 mL) at -78 °C. The reaction mixture was warmed to room temperature slowly. After stirring at room temperature for 2 hours, the mixture was quenched by adding water and the organic layer was extracted with DCM, dried over Na₂SO₄ and purified by silica gel column chromatography to give the title compound.

To the above obtained compound in THF (2 mL) was added HCI (6 M, 2 mL) and the mixture was stirred for 1.5 h. Again, HCI (6 M, 1 mL) was added and the mixture was stirred for another 1 h. This process was continued until the complete consumption of starting compound was observed (UHPLC). After completion of the reaction, the mixture was quenched by slowly adding water and sat. NaHCO₃. The product was extracted with EtOAc, dried over Na₂SO₄ and concentrated to give the pure product (72% yield for 2 steps). **¹H NMR (400 MHz, DMSO-*d*₆)** δ 9.60 (s, 1H), 8.88 (s, 2H), 6.64 (s, 1H), 6.51 (s, 1H), 5.11 (s, 1H), 3.96 (s, 2H), 3.75 - 3.35 (m, 8H), 1.37 (s, 9H). **¹³C NMR (101 MHz, dmso)** δ 168.69, 164.44, 161.14, 159.44, 143.32, 137.02, 135.89, 129.50, 118.83, 116.83, 110.18, 45.18, 42.21, 40.83, 40.63, 40.42, 40.21, 40.00, 39.79, 39.58, 35.74, 31.11, 30.55; **HRMS (ESI):** m/z calcd for C₂₁H₂₆O₃N₅Cl₂ [M+H]⁺ : 466.1407, found: 466.1421.

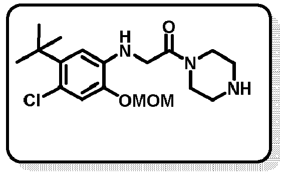

**2-((5-(*tert*-butyl)-4-chloro-2-(methoxymethoxy)phenyl)amino)-1-(piperazin-1-yl)ethan-1-one:** To a solution of benzyl 4-((5-(*tert*-butyl)-4-chloro-2-(methoxy-methoxy)phenyl)glycyl)piperazine-1-carboxylate (5.00 g, 9.92 mmol) in MeOH (40 mL) was added slowly portion wise 10% Pd/C (1.00 g), and the mixture was stirred under H₂ atmosphere for 15 h. The mixture was filtered through a pad of celite and the celite pad was washed with EtOAc. The filtrate was concentrated *in vacuo* to provide the pure product in a quantitative yield. **¹H NMR (400 MHz, Chloroform-*d*)** δ 7.02 (s, 1H), 6.51 (s, 1H), 5.31 (s, 1H), 5.16 (s, 2H), 3.88 (s, 2H), 3.65 (t, *J* = 5.2 Hz, 2H), 3.48 (s, 3H), 3.44 (t, *J* = 5.0 Hz, 2H), 2.89 (dt, *J* = 16.0, 5.2 Hz, 4H), 1.77 (s, 1H), 1.44 (s, 9H). **¹³C NMR (101 MHz, cdcl₃)** δ 167.37, 142.92, 140.04, 136.24, 120.50, 117.56, 109.50, 95.24, 77.32, 77.00, 76.68, 56.19, 46.18, 45.80, 45.58, 44.94, 43.15, 35.58, 29.80; **HRMS (ESI):** m/z calcd for C₁₈H₂₉O₃N₃Cl [M+H]⁺ : 370.1892, found: 370.1904.

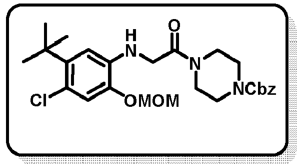

**benzyl 4-((5-(*tert*-butyl)-4-chloro-2-(methoxymethoxy)phenyl)glycyl)-piperazine-1-carboxylate:** To a solution containing 5-(*tert*-butyl)-4-chloro-2-(methoxymethoxy)aniline (5.00 g, 20.51 mmol) in DMF was added oven dried K₂CO₃ (5.67 g, 41.03 mmol) at room temperature. The resulting reaction mixture was stirred for 30 min, bromide (20.51 mmol) was added and continued to stir at 90 °C for 18 h. The mixture was poured into water and extracted with ethyl acetate. The organic layer was combined, washed with water and brine, dried over anhydrous Na₂SO₄, and evaporated. The desired product was obtained by column chromatography (yield 68%). **¹H NMR (400 MHz, Chloroform-d)** δ 7.40 - 7.28 (m, 5H), 7.02 (s, 1H), 6.52 (s, 1H), 5.27 (s, 1H), 5.15 (s, 4H), 3.90 (s, 2H), 3.72 - 3.43 (m, 8H), 3.48 (s, 3H), 1.45 (s, 9H). **¹³C NMR (101 MHz, cdcl₃)** δ 167.61, 154.94, 142.82, 139.98, 136.15, 135.99, 128.47, 128.14, 127.93, 120.60, 117.49, 109.48, 95.15, 77.32, 77.00, 76.68, 67.46, 56.10, 45.03, 44.07, 43.50, 41.67, 35.50, 29.71; **HRMS (ESI):** m/z calcd for C₂₆H₃₅O₅N₃Cl [M+H]⁺ : 504.2260, found: 504.2270.

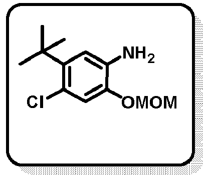

**5-(*tert*-butyl)-4-chloro-2-(methoxymethoxy)aniline:** To N-(5-(tert-butyl)-4-chloro-2-(methoxymethoxy)phenyl)acetamide in EtOH (15 mL) was added KOH (4.0 M, 20 mL) and the resulting reaction mixture was heated at 90 °C for 15 h. After completion of the reaction, the mixture was diluted to pH = 7, extracted with EtOAc, dried over Na₂SO₄ and concentrated. The desired product was obtained after silica gel column chromatography (yield 90%). **¹H NMR (400 MHz, Chloroform-*d*)** δ 7.02 (s, 1H), 6.78 (s, 1H), 5.14 (s, 2H), 3.78 (s, 2H), 3.49 (s, 3H), 1.42 (s, 9H). **¹³C NMR (101 MHz, cdcl₃)** δ 142.91, 140.35, 134.81, 121.87, 118.24, 114.59, 95.26, 77.32, 77.00, 76.68, 56.14, 35.37, 29.72; **HRMS (ESI):** m/z calcd for C₁₂H₁₉O₂NCl [M+H]⁺ : 244.1099, found: 244.1103.

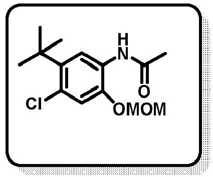

***N*-(5-(*tert*-butyl)-4-chloro-2-(methoxymethoxy)phenyl)acetamide:** To known compound *N*-(5-acetyl-2-hydroxyphenyl)acetamide (5 g, 1 eq) in DCM (30 mL) DIPEA (1.5 eq) and at 0 °C MOMCI (1.2 eq) were added. The resulting reaction mixture was left at rt overnight. After completion of the reaction, the reaction mixture was poured into water (50 mL) and the organic layer was extracted with DCM. The combined organic layers were washed with brine, dried over Na₂SO₄ and concentrated *in vacuo.* The residue was purified by silica gel column chromatography to afford the desired product in quantitative yield.
To the above obtained product (2.00 g, 8.02 mmol) in DCM (20 mL) at room temperature was added NCS (8.02 mmol, 1 eq) and the resulting reaction mixture was stirred for 12 h. After completion of the reaction (UHPLC), the mixture was concentrated and purified by silica gel column to give the title compound in quantitative yield. **¹H NMR (400 MHz, Chloroform-*d*)** δ 8.53 (s, 1H), 7.61 (s, 1H), 7.11 (s, 1H), 5.18 (s, 2H), 3.49 (s, 3H), 2.19(s, 3H), 1.45 (s, 9H).

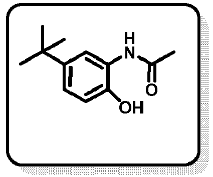

***N*-(5-(*tert*-butyl)-2-hydroxyphenyl)acetamide:** To a solution of 2-amino-4-(tert-butyl)phenol (5.0 g, 1.0 equiv) in ethyl acetate (50 mL) at 0 °C, acetic anhydride (2.00 equiv) was added slowly over 15 min. The mixture was allowed to warm to room temperature over 5 h during which a solid started to precipitate out of the solution. The mixture was concentrated under reduced pressure to leave -10% of the initial ethyl acetate. To this slurry hexanes (50 mL) were added, stirred for 10 min and filtered. The solid residue was washed with hexanes (15 mL) and dried to give the pure product. **¹H NMR (400 MHz, Chloroform-*d*)** δ 8.67 (s, 1H), 7.76 (s, 1H), 7.14 (dd, *J* = 8.5, 2.4 Hz, 1H), 6.99 (d, *J* = 2.4 Hz, 1H), 6.94 (d, *J* = 8.5 Hz, 1H), 2.24 (s, 3H), 1.25 (s, 9H).

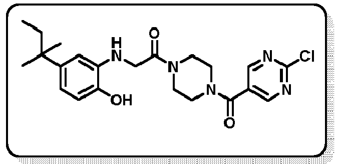

**1-(4-(2-chloropyrimidine-5-carbonyl)piperazin-1-yl)-2-((2-hydroxy-5-(tert-pentyl)phenyl)amino)ethan-1-one:** Synthesized according to the procedure described above. **¹H NMR (400 MHz, DMSO-*d*₆)** δ 9.05 (s, 1H), 8.88 (s, 2H), 6.55 (d, *J* = 8.0 Hz, 1H), 6.47 (s, 1H), 6.35 (dd, *J* = 8.0, 2.1 Hz, 1H), 5.04 (s, 1H), 3.92 (s, 2H), 3.54 (dt, *J* = 61.7, 25.6 Hz, 8H), 1.58 - 1.49 (m, 2H), 1.16 (s, 6H), 0.63 - 0.57 (m, 3H). **¹³C NMR (101 MHz, dmso)** δ 168.95, 164.43, 161.14, 159.45, 142.46, 140.76, 136.83, 129.51, 113.96, 113.28, 109.10, 45.47, 40.83, 40.62, 40.41, 40.20, 40.00, 39.79, 39.58, 37.70, 37.05, 31.11, 29.30, 29.22, 9.86; **HRMS (ESI):** m/z calcd. for C₂₂H₂₉O₃N₅Cl [M+H]⁺ : 446.1953, found: 446.1966.

The compounds in the following table were prepared similar to the procedure described in the previous example. The purification of the crude product was performed by flash silica gel column chromatography with MeOH and CH₂Cl₂ as eluents, by reverse phase RP-HPLC (column: Cl8), using H₂O (0.1%TFA) and ACN (0.1 %TFA) as eluents or by precipitation.

| **No.** | **Structure** | **MW** | **[M+H]+** |
|---|---|---|---|
| **1** | 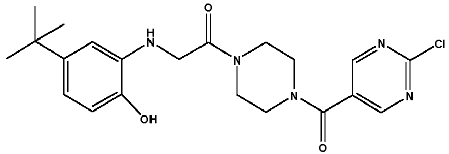 | **431** | **432** |
| **2** | 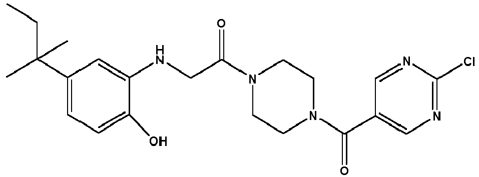 | **445** | **446** |
| **3** | 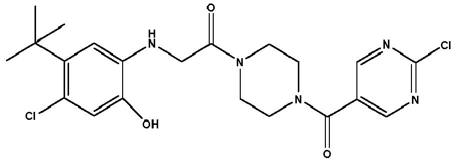 | **465** | **466** |
| **4** | 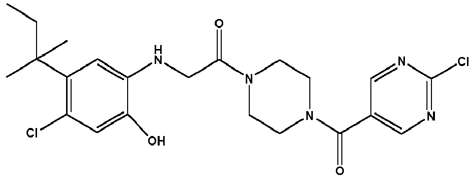 | **479** | **480** |
| **5** | 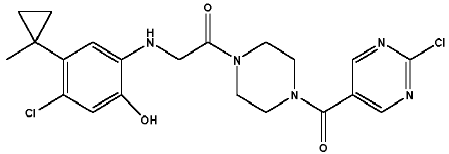 | **463** | **464** |
| **6** | 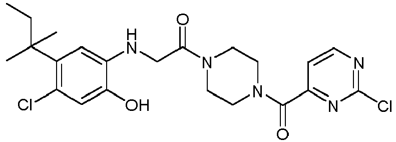 | **479** | **480** |
| **7** | 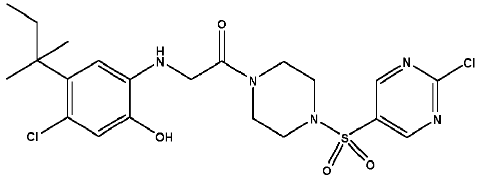 | **515** | **516** |
| **8** | 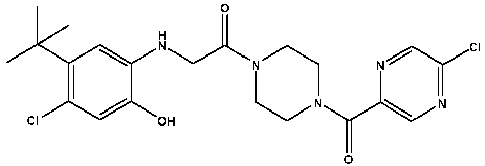 | **465** | **466** |
| **9** | 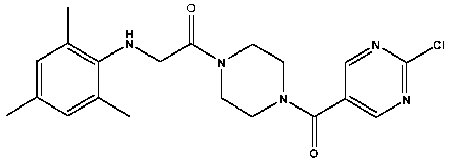 | **401** | **402** |

### B. Hydrolysis Stability

Compound **4** was dissolved in HEPES (4-(2-hydroxyethyl)-1-piperazineethanesulfonic acid) buffer (pH 7.5) at 37 °C for 24 hours. The LC-MS analysis revealed that the tested compounds were stable against hydrolysis **(see** **Figure 3****).**

### C. Biochemical experiments

### Live cell imaging experiments

All cell lines (BxPC-3, Panc-I, Panc-TUI, MIA PaCa-2) were cultured in Dulbecco's modified Eagle's medium (DMEM, PAN Biotech) supplemented with 10% v/v fetaal bovine serum (FBS, Gibco) and 100 U/mL penicillin, 100 µg/mL streptomycin (10000 U/mL penicillin, 1 mg/mL streptomycin, PAN Biotech), 25 mM glucose, 4 mM glutamine, and 1 mM sodium pyruvate (100 mM, PAN Biotech). For live cell imaging experiments the respective cell lines where seeded 24 hours before the experiment in a 96 well format (5,000 cells/well). For growth curves the media was exchanged for compound containing DMEM media without any washing step. The cell growth was monitored using the IncuCyte ZOOM or the IncuCyte S3 (Essenbioscience, Satorius). The quantification of the cellular confluence was conducted using the IncuCyte ZOOM 2016B or IncuCyte S3 2017A software (EssenBioscience, Satorius). Graphs were prepared with GraphPad Prism 7. All experiments were performed in at least 3 biological replicates (n=3, N=3).

### Tethering assay

Protein was incubated with the compounds in 20 mM HEPES, pH 7.5, 150 mM NaCl at room temperature for the specific times. For MALDI experiments, a saturated solution of sinapic acid (Aldrich) in H₂O (0.1% TFA) / ACN (2:1) was used as matrix. 1 µL of the samples was mixed with 5 µL of matrix, and 1 µL of this mixture was placed on an MTP 384 ground steel target plate and dried in air. Mass spectra were obtained over the *m*/*z* range 17.000-21.000 using a Bruker UltrafleXtreme TOF/TOF mass spectrometer.
The labeling efficiency of the compounds was classified according to the percentage of covalent adduct formation after 30 min, 24 h and 40 h into the following ranges:

| | |
|---|---|
| covalent labeling > 90% | +++ |
| 50 < covalent labeling ≤ 90 | ++ |
| 5 < covalent labeling ≤ 50 | + |
| covalent labeling not observed | - |

**Table 1. Covalent adduct formation for compounds of general formula (I) as determined by MALDI.**

| **Compound** | **30 min** | **24h** | **48h** |
|---|---|---|---|
| **1** | **+++** | **+++** | **+++** |
| **2** | **+++** | **+++** | **+++** |
| **3** | **+++** | **+++** | **+++** |
| **4** | **+++** | **+++** | **+++** |
| **5** | **+++** | **+++** | **+++** |
| **6** | **+** | **++** | **+++** |
| **7** | **+++** | **+++** | **+++** |
| **8** | **+** | **++** | **+++** |
| **9** | **+++** | **+++** | **+++** |

## Claims

1. A compound of general formula (I): 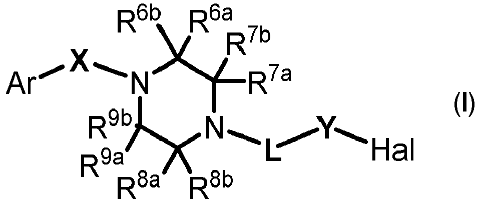 wherein
Ar represents 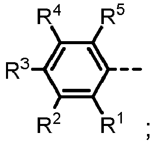 **R¹** - **R⁵** represent independently of each other -H, -F, -CI, -CN, -CF₃, -OCF₃, -CH₃, -C₂H₅, -C₃H₇, -C₄H₉, -CH(CH₃)₂, -CH₂CH(CH₃)₂, -CH(CH₃)-C₂H₅, -C(CH₃)₃, -C(CH₃)R¹⁰R¹¹, -*cyclo*-C₃H₅, -*cyclo*-C₄H₇, *-cyclo*-C₅H₉, -CH₂-cyclo-C₃H₅, -Ph, -OH, 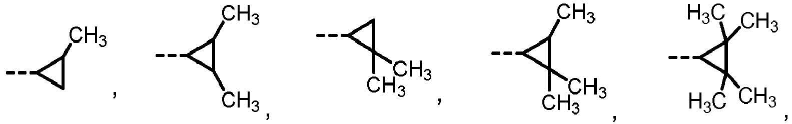 -OCH₃, -OC₂H₅, -OC₃H₇, -OC₄H₉, -OCH(CH₃)₂, -OCH₂CH(CH₃)₂, -OCH(CH₃)-C₂H₅, -OC(CH₃)₃, -O-*cyclo*-C₃H₅, -OCH₂-*cyclo*-C₃H₅, -NH₂, -NH(CH₃), -NH(C₂H₅), -N(CH₃)₂ or -N(C₂H₅)₂;
**R^{6a}** - **R^{9a}** and **R^{6b}** - **R^{9b}** represent independently of each other -H, -OH, -CN, -CH₃, -C₂H₅, -C₃H₇, -CH(CH₃)₂, -CF₃, -OCH₃, -OC₂H₅, -OC₃H₇, -OCH(CH₃)₂, -OCF₃, -OCH₂OCH₃, -O-cyclo-C₃H₅, -OCH₂-cyclo-C₃H₅, -COOCH₃, -COOC₂H₅, -OOC-CH₃, -OOC-CF₃, -OOC-C₂H₅, -OOC-C(CH₃)₃, -NH₂, -NHCH₃, -NHC₂H₅, -NHCH(CH₃)₂, -N(CH₃)₂, -N(C₂H₅)₂, -CH₂NH₂, -CH₂NHCH₃, -CH₂NHC₂H₅, -CH₂N(CH₃)₂, -CH₂N(C₂H₅)₂, -NHCOCH₃, -NHCOCF₃, -CONH₂, -CONHCH₃, -CONHC₂H₅, -CON(CH₃)₂, -CON(C₂H₅)₂, -SO₂NH₂, -SO₂NHCH₃, -SO₂NHC₂H₅, -SO₂N(CH₃)₂, -SO₂N(C₂H₅)₂, -NHSO₂CH₃, -NHSO₂CF₃ or -NHSO₂C₂H₅; or
**R^{6a}** and **R^{7a}** join to form a 5-membered or 6-membered carbocyclic or heterocyclic ring, or **R^{6b}** and **R^{7b}** join to form a 5-membered or 6-membered carbocyclic or heterocyclic ring, or **R^{8a}** and **R^{9a}** join to form a 5-membered or 6-membered carbocyclic or heterocyclic ring, or **R^{8b}** and **R^{9b}** join to form a 5-membered or 6-membered carbocyclic or heterocyclic ring;
**R¹⁰** and **R¹¹** represent independently of each other -CH₃, -CH₂CH₃, -CH₂CH₂CH₃, -CH(CH₃)₂, or **R¹⁰** and **R¹¹** together with carbon atom to which they are attached form a cyclopropane, cyclobutane or cyclopentane ring;
L represents -C(O)- or -SO₂- ;
X represents -NH-CH₂-CO- or -O-CH₂-CO;
Y represents 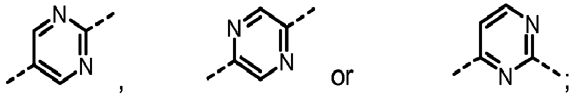 **Hal** represents -CI, -Br or -F;
or pharmaceutically acceptable salts thereof.

2. The compound according to claim 1, wherein Ar represents 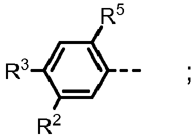 **R²** represents -C(CH₃)R¹⁰R¹¹, -*cyclo*-C₃H₅, -*cyclo*-C₄H₇, -*cyclo*-C₅H₉, 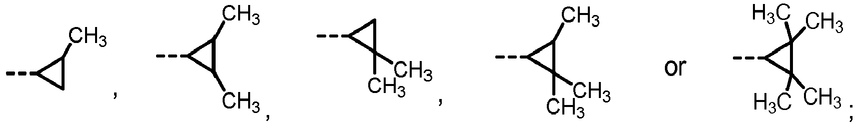 **R³** and **R⁵** represent independently of each other -H, -F, -CI, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and
**R¹⁰** and **R¹¹** have the meanings as defined in claim 1;
or
Ar represents 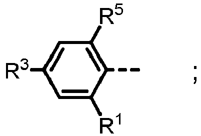 **R¹, R³,** and **R⁵** represent independently of each other -H, -F, -CI, -CN, -CF₃, -OCF₃, -CH₃, -C₂H₅, -C₃H₇, -CH(CH₃)₂, -OH, -OCH₃, -OC₂H₅, -NH₂, -NH(CH₃), -NH(C₂H₅), -N(CH₃)₂, -N(C₂H₅)₂; and **R¹⁰** and **R¹¹** have the meanings as defined in claim 1.

3. The compound according to claim 2, wherein
Ar represents 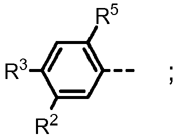 **R²** represents -C(CH₃)₃, -C(CH₃)₂CH₂CH₃ or 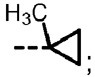 **R³** represents -H or -Cl and **R⁵** represents -OH; **or**
Ar represents 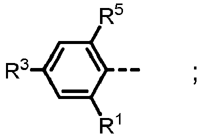 **R¹, R³** and **R⁵** represent independently of each other -H, -CH₃, -CH₂CH₃, -CH(CH₃)₂ or -CH₂CH₂CH₃.

4. The compound according to any one of claims 1 - 3, wherein **R^{6a}** - **R^{9a}** and **R^{6b}** - **R^{9b}** represent -H.

5. The compound according to any one of claims 1 - 4, wherein **Hal** represents -Cl.

6. The compound according to any one of claims 1 - 5, wherein **X** represents -NH-CH₂-CO- .

7. The compound according to claim 1, selected from the group consisting of:
2-((5-(*tert*-butyl)-2-hydroxyphenyl)amino)-1-(4-(2-chloropyrimidine-5-carbonyl)piperazin-1-yl)ethan-1-one;
1-(4-(2-chloropyrimidine-5-carbonyl)piperazin-1-yl)-2-((2-hydroxy-5-(*tert-*pentyl)phenyl)amino)ethan-1-one;
2-((5-(*tert*-butyl)-4-chloro-2-hydroxyphenyl)amino)-1-(4-(2-chloropyrimidine-5-carbonyl)piperazin-1-yl)ethan-1-one;
2-((4-chloro-2-hydroxy-5-(*tert*-pentyl)phenyl)amino)-1-(4-(2-chloropyrimidine-5-carbonyl)piperazin-1-yl)ethan-1-one;
2-((4-chloro-2-hydroxy-5-(1-methylcyclopropyl)phenyl)amino)-1-(4-(2-chloropyrimidine-5-carbonyl)piperazin-1-yl)ethan-1-one;
2-((4-chloro-2-hydroxy-5-(*tert*-pentyl)phenyl)amino)-1-(4-(2-chloropyrimidine-4-carbonyl)piperazin-1-yl)ethan-1-one;
2-((4-chloro-2-hydroxy-5-(*tert*-pentyl)phenyl)amino)-1-(4-((2-chloropyrimidin-5-yl)sulfonyl)piperazin-1-yl)ethan-1-one;
2-((5-(*tert*-butyl)-4-chloro-2-hydroxyphenyl)amino)-1-(4-(5-chloropyrazine-2-carbonyl)piperazin-1-yl)ethan-1-one;
1-(4-(2-chloropyrimidine-5-carbonyl)piperazin-1-yl)-2-(mesitylamino)ethan-1-one.

8. A compound according to any one of claims 1 - 7 for use as a pharmaceutically active agent in medicine.

9. A compound according to any one of claims 1 - 7 for use in the treatment or prophylaxis of proliferative diseases, tumors or cancer.

10. The compound for use according to claim 9, wherein the proliferative disease, tumor or cancer is selected from the group comprising:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

11. The compound for use according to claim 9, wherein the proliferative disease, tumor or cancer is associated and/or caused by KRas G12C mutant.

12. A pharmaceutical composition comprising at least one compound according to any one of claims 1 to 7 together with at least one pharmaceutically acceptable carrier, excipient and/or diluent.

13. The pharmaceutical composition according to claim 12 for use in the treatment or prophylaxis of proliferative diseases, tumors or cancer.

14. The pharmaceutical composition for use according to claim 13, wherein the proliferative disease, tumor or cancer is selected from the group comprising: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

15. The pharmaceutical composition for use according to claim 13, wherein the proliferative disease, tumor or cancer is associated and/or caused by KRas G12C mutant.
